# EUROPEAN PATENT APPLICATION

(11) **EP 2 886 163 A1**
(43) Date of publication of application: **24.06.2015**
(21) Application number: 14196665.5
(22) Date of filing: 20.12.2011
(51) Int. Cl.: A61Q 19/08

(54) **Compounds with Anti-aging activities**

(62) Divisional of application: 11797020.2
(71) Applicant: Oriflame Research and Development Ltd., Bray, Co. Wicklow (IE)
(72) Inventor: Gillbro, Johanna Maria, 11736 Stockholm (SE); Mavon, Alain Robert Pierre, 11264 Stockholm (SE); Duracher, Lucie, 11761 Stockholm (SE); Klack, Anke, 11244 Stockholm (SE); Cattley, Kevin, Wexford (IE)
(74) Representative: Moore, Michael Richard

(57) **Abstract**

Dermatological use of N-acetylaspartic acids and combinations of N-acetylaspartic acids and at least one of a compound selected from the group consisting of: gibberellic acids, naringenins, β-aescin, arachadonic acid, quercetin, vitexin and docosahexaenoic acid (ethyl ester). Suitably, the use is in the treatment of, and/or prevention of, at least one sign of skin ageing or at least one sign of a skin damage condition associated with ageing, wherein the sign of skin ageing or skin damage is present on skin of the face, body or the scalp of a subject.

## Description

### Field of the Invention:

The invention relates to compounds, or plant extracts comprising these compounds, which combat the effects of skin aging and/or provide anti-aging benefits. More particularly, the invention relates to compounds having retinoic acid like effects on the skin, but without producing harsh effects and skin irritation caused by retinoic acid usage.

### Background to the Invention:

Aging is a multifactorial phenomenon. The aging of the skin is mainly the result of one's genetic predisposition (known as chronological aging) and one's physiological reaction to environmental stresses (known as actinic aging). Actinic aging seems to be skin specific and is defined as the effect of the external environment on the skin's biological response. The skin response to actinic aging, which may be caused by sun and pollution exposure, as well as smoking, is typically associated with a lack of normal hydration, apparition of telangiectasia (spider veins), sagging of the skin, and the appearance of fine lines and wrinkles. In addition, temporary or even lasting changes to the skin can occur with age, such as hormonal associated acne, greasy or dry skin, keratoses, rosacea, light sensitivity or inflammatory erythema. The colour of the skin is another large contributor to a person's appearance and an uneven skin tone with visible or tactile discontinuities and puffiness are perceived as older unhealthier looking skin. Moreover, many people are concerned with the degree of pigmentation of their skin and may wish to reduce darkening, or even lighten their 'natural' skin colour. The process of keratinocyte differentiation and desquamation are essential for both intact barrier function and for healthy looking skin. As we age, these functions decrease resulting in impaired barrier function, which leads to loss of moisture and ultimately to dry skin, but also increased susceptibility of the skin to external insults such as infection and photo damage. Sun-exposure of the skin causes thickening of the epidermis. This could be a result of senescent keratinocytes and melanocytes surviving for a prolonged time, possibly due to defective desquamation. Stimulating keratinocytes to maintain the balance of proliferating and differentiating keratinocytes, improves both barrier function and the appearance of the skin, thereby providing healthy younger looking skin. Mechanical methods can be used to stimulate desquamation. However, these methods commonly also have a proteolytic and/or keratinolytic activity, resulting in irritation of the skin. Thus, there is a need for compounds that stimulate desquamation without having the disadvantage of damaging other aspects of the skin. A further feature of aging skin is impaired or slowed wound healing. Wound healing is a complex process involving migration, proliferation, differentiation and infiltration of several different cell types and the production of numerous growth factors, cytokines and tissue restoring molecules resulting in the synthesis of new tissue and wound closure. Retinoic acid is the gold standard anti-aging ingredient, but is not without uncomfortable side effects and regulatory restrictions on its use. Alternative retinoid-like actives have similar, but fewer, restrictions. A side effect of topical retinoid use is skin irritation. Irritated skin is characterized by redness, dryness and flaking at the treated site. At the histological level, a perivascular accumulation of mononuclear cells is observed, with neutrophils and monocytes scattered throughout the dermis and occasional micro-abscesses in the dermis or epidermis. All-trans retinol (vitamin A), the parent compound of retinoic acid, tends to be less irritating than retinoic acid in most cases, but even with this agent, significant irritation is observed in many individuals. Irritation is a major cause of non-compliance among retinoid users. In addition, excessive irritation may counteract the beneficial effects of topical use. Due to the high level of skin irritation arising from use of retinoic acid, there is a high need for alternative compounds having similar skin beneficial efficacy as retinoic acid, but without the associated skin irritation issues. There remains a need to identify compounds which will deliver anti-aging benefits, which address the consumer desire to look younger and which avoid the adverse effects of retinoid administration.

### Statements of the Invention:

In one aspect of the invention there is provided non-therapeutic cosmetic or dermatological use of at least one of a compound selected from the group consisting of: gibberellic acids, naringenins, N-acetylaspartic acids, β-aescin, arachadonic acid, quercetin, vitexin and docosahexaenoic acid (ethyl ester).

The inventors have discovered that these compounds have retinoic acid-mimicking properties, and produce effects on the skin, which are similar to the effects produced by retinoic acid. Accordingly, the compounds can be used in place of retinoic acid or its derivatives in skin care or dermatological compositions. Accordingly, the inventors have provided a series of compounds, which may be used in compositions for cosmetic use in place of retinoic acid which may have been used. In particular, such compositions may be used to protect against, and/or alleviate signs of aging. The compounds of the invention are suitable for use in the treatment of, and/or prevention of, at least one sign of skin ageing or at least one sign of a skin damage condition associated with ageing, wherein the sign of skin ageing or skin damage is present on skin of the face, body or the scalp of a subject. By the term "skin", it is intended to cover skin found on the face, the body and the scalp.

The compounds of the invention have been advantageously found to be non-irritating to the skin. Accordingly, the compounds of the invention can be applied to skin in a substantially pure form, or can used as plant extract, alone or as part of a composition or formulation, without causing irritation. This is advantageous over use of retinoic acid or its derivatives, as unlike, retinoic acid or its derivatives; the compounds of the invention do not irritate the skin.

The skilled person will appreciate that the compounds of the invention may therefore be used in pure or semi-pure form, or as crude extracts of natural products, such as plant extracts, obtained from any part of the plant, including roots, stems, leaves, seeds, flower and fruit.

By *"plant extract"* it is meant a preparation in liquid, semi-solid or solid form, obtained from plant or herbal material which is usually in the dry state. Different types of extracts may be distinguished. Standardised extracts are adjusted within an acceptable tolerance to a given a content of constituents with a known efficacy of activity; standardisation is achieved by adjustment of the extract with inert material or by blending batches of extracts. Quantified extracts are adjusted to a defined range of constituents; adjustments are made by blending batches of extracts. Other extracts are essentially defined by their production process (state of raw material to be extracted, solvent, extraction conditions) and their specifications. Extracts are prepared by suitable methods using ethanol or other suitable solvents including supercritical gas and liquids. Different batches of herbal extraction material may be blended prior to extraction. The herbal matter may undergo a preliminary treatment, for example, inactivation of enzymes, grinding ordefatting. In addition unwanted matter may be removed after extraction.

The skilled person will appreciate that any individual one of these compounds may be used on its own or any combination of two or more of the compounds can equally well be used for additive effects or effect on more than one sign of aging.

Particularly preferred compounds of the invention for the uses described herein, are selected from the group consisting of: naringenins, gibberellic acids and N-acetylaspartic acids, as defined below. These particular compounds have been found to easily penetrate the skin, without irritation. A particularly preferred compound is naringenin. A further particularly, preferred compound is gibberellic acid. A further still particularly, preferred compound is N-acetylaspartic acid.

N-acetyl aspartic acid is an osmolyte and a precursor for other biomolecules in the central nervous system of humans. N-Acetyl-L-aspartic acid ((2S)-2-Acetamidobutanedioic acid) as used herein is one example of the N-Acetyl-L-aspartic acid type compounds (N-acetyl aspartic acids) of the invention having the following general structure: 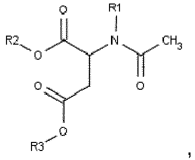 wherein R₁ is hydrogen, or an unsubstituted, branched or unbranched, unsaturated or saturated C₁-C₆alkyl, allyl, or aryl group; R₂ is hydrogen, or a substituted or unsubstituted, branched or unbranched, unsaturated or saturated C₁-C₆ alkyl, allyl or aryl group; R₃ is hydrogen, or a substituted or unsubstituted, branched or unbranched, unsaturated or saturated C₁-C₆ alkyl, allyl or aryl group.

The skilled person will appreciate that optical rotational isomers, for example, N-Acetyl-L-Aspartic acid, N-acetyl-D,L-aspartic acid and N-Acetyl-D-aspartic acid may also be used, however, the compound N-acetyl-L-aspartic acid is the preferred compound of the invention.

Naringenin is a flavanone compound widely found in citrus fruit (Citrus var.) such as, but not limited to, grapefruit (Citrus Paradisi), orange (Citrus sinensis); and also tomato (Solanum Lycopersicum). Naringenin (S)-2,3-Dihydro-5,7-dihydroxy- 2-(4-hydroxyphenyl)- 4H-1-benzopyran-4-one) as used herein is one example of suitable naringenins, which can be represented by the general formula: 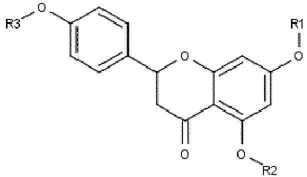 wherein R1 is -H, a glycoside or a substituted or unsubstituted, branched or unbranched, unsaturated or saturated, C₁-C₆ alkyl, allyl or aryl group. Where R1 is glycoside, a sugar group (monosaccharide) or group of sugars (oiligosaccharide) is bonded through an anomeric carbon to oxygen on the naringenin structure, via an O-glycoside bond; R2 is -H, a glycoside, substituted or unsubstituted, branched or unbranched, unsaturated or saturated C1-C6 alkyl, allyl or aryl group. Where R2 is glycoside, a sugar group (monosaccharide) or group of sugars (oiligosacchande) is bonded through an anomeric carbon to oxygen on the naringenin structure, via an O-glycoside bond; R3 is -H, glycoside, substituted or unsubstituted, branched or unbranched, unsaturated or saturated ,C1-C6 alkyl, allyl or aryl group. Where R3 is glycoside, a sugar group (monosaccharide) or group of sugars (oiligosacchande) is bonded through an anomeric carbon to oxygen on the naringenin structure, via an O-glycoside bond. Especially preferred are compounds in which R1= H, R2 = H and R3 = H. The term "Naringenin" as used herein is intended to cover the Naringenin derivatives as described above.

Gibberellic acid is ubiquitous in plants and plays a role in stimulating rapid elongation growth, inducing mitotic division in the leaves of some plants, and increasing seed germination rate. Gibberellic acid (3S,3aS,4S,4aS,7S,9aR,9bR,12S)-7,12-dihydroxy-3-methyl-6-methylene-2-oxoperhydro-4a,7-methano-9b,3-propenoazuleno[1,2-b]furan-4-carboxylic acid) is one example of gibberellic acids falling with the scope of the present invention, and can be represented by the general formula: 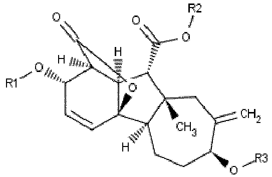 wherein R1 is selected from, -H, a glycoside, substituted or unsubstituted, branched or unbranched, unsaturated or saturated C1-C6 alkyl, allyl or aryl group (where R1 is glycoside, a sugar group (monosaccharide) orgroup of sugars (oiligosacchande) is bonded through an anomeric carbon to oxygen on the naringenin structure, via an O-glycoside bond);R2 is selected from -H, substituted or unsubstituted, branched or unbranched, unsaturated or saturated C1-C6 alkyl, allyl or aryl group; R3 is selected from -H, glycoside, substituted or unsubstituted, branched or unbranched, unsaturated or saturated C1-C6 alkyl, allyl or aryl group. Where R1 is glycoside, a sugar group (monosaccharide) or group of sugars (oiligosacchande) is bonded through an anomeric carbon to oxygen on the naringenin structure, via an O-glycoside bond. Especially preferred are compounds in which R1= H, R2 = H and R3 = H.

The term "gibberellic acid" as used herein is intended to cover the gibberellic acid derivatives as described above.

β-aescin is a saponin and can be found in extracts of horse chestnut (Aesculus Hippocastanum). Accordingly, extracts of horse chestnut comprising β-aescin can be used in the methods of the invention.

Docosahexaenoic acid (ethyl ester) is an omega-3 fatty acid ester and is contained in fish oils, chloroplast containing microalgae and cyanobacteria, like spirulina. Accordingly, fish oils and spirulina, comprising docosahexaenoic acid (ethyl ester) can be used in the methods of the invention.

Arachidonic acid is an omega-6 fatty acid and is contained within sources such as olive oil (Olea europaea), canola oil (Brassica napus), sesame oil (Sesamum indicum), schizochytrium algae, black cumin seed oil (Nigella sativa), blackcurrant seed oil (Ribes nigrum), cashew nut oil (Anacardium L.), bilberry seed oil (Vaccinum myrtillus). Accordingly, extracts from these sources, comprising arachidonic acid can be used in the methods of the invention.

Quercetin is a flavanol derivative and is contained within plant sources such as black and green tea (Camellia sinensis), capers (Capparis spinosa), apple (Malus domestica), onion (Allium cepa), grapes (Vitis vinifera), citrus fruit (Citrus var.), tomato (Solanum Lycopersicum), broccoli (Brassica Oleracea), raspberry (Rubus var.), plamt extracts from the vaccinium genus such as Vaccinum myrtillus, Vaccinum vitis ideae, Vaccinum Oxyoccus, red chokeberry (Aronia Arbutifolia), rowanberry (Sorbus var), sea buckthom (Hippophae L.), crowberry (Empetrum var.) Accordingly, extracts from these sources, comprising quercetin can be used in the methods of the invention.

Vitexin is a flavone derivative and is contained within plant sources such as passion flower (Passiflora), chaste berry (Vitex Agnus Castus), bamboo leaves (Phyllostachys nigra). Accordingly, extracts from these sources, comprising vitexin can be used in the methods of the invention.

Accordingly, the invention pertains to use of at least one of the above-mentioned compounds in the treatment and/or prevention and/or cure at least one sign of skin ageing or of skin damage condition associated with ageing. Preferably, the compounds are used directly on skin of the face, body or the scalp of a subject or may be applied thereto as part of a suitable delivery composition or formulation. Extracts or oils comprising these compounds may also be used directly or as part of a cosmetic or dermatological composition. Suitably, the at least one compound may be used in accordance with the invention wherein the at least one sign of skin ageing or the skin damage condition is produced by intrinsic biological aging (natural ageing) or photo-induced ageing (actinic ageing), caused by, for example, exposure to UV light, pollution or stress. The skilled person will appreciate that the treatable and/or preventable signs of aging which benefit from application of the at least one compound of the invention include wrinkles, skin with fine lines, wizened skin, lack of skin elasticity, lack of skin tone, thinned skin, sagging skin, skin suffering from degradation of collagen fibres, flaccid skin, sagging skin, and skin suffering from internal degradation. A particularly preferred compound for use in at least one of the above applications is naringenin. A further particularly preferred compound for use in at least one of the above applications is gibberellic acid. A further still particularly preferred compound for use in least one of the above applications compound is N-acetylaspartic acid.

The at least one compound of the invention may be used to treat and/or prevent simultaneously all of these signs, any one of these signs or indeed any combination of two or more of these signs of aging. The skilled person will also appreciate that other recognised signs of aging not described herein but treatable by retinoic acid or its derivatives can benefit from application of the at least one compounds of the invention.

The treatable and/or preventable skin damage condition which benefits from the application of the at least one compound of the invention involves at least one of: inflammation, redness, blotchiness, puffy eyes or dark circles, and/or or skin pigmentation disorders.

The at least one compound of the invention may be used for at least one of skin lightening, for improving wound healing, for promoting evenness of skin tone by reducing skin redness, blotchiness or inflammation, for lightening (whitening) the colour of the skin or scalp, for promoting skin regeneration to produce more homogenous, firmer, more toned and more elastic skin, for promoting cell longevity, for promoting skin brightness, for promoting skin texture and tone uniformity and/orfor reducing the appearance of skin pigmentation, and for the treatment or prevention of ulcerated areas or areas of cutaneous stress or damage brought about by exposure to UV or exposure to an irritant product, and/or treating acne or other skin blemishes. In a preferred embodiment, the at least one of the compound of the invention may be formulated into a cosmetically acceptable composition for topical application to at least one of the skin, body and/or scalp. A particularly preferred compound for use in at least one of the above applications is naringenin. A further particularly preferred compound for use in at least one of the above applications is gibberellic acid. A further still particularly preferred compound for use in least one of the above applications compound is N-acetylaspartic acid.

Suitably, the cosmetic or dermatological compositions of the invention may comprise, in a physiologically acceptable medium, at least one of, or a combination of, compound(s) selected from the group consisting of: gibberellic acid, naringenin, N-acetylaspartic acid, β-aescin, arachadonic acid, quercetin, vitexin and docosahexaenoic acid (ethyl ester). Particularly preferred compounds of the invention for the described use are naringenin, gibberellic acid or N-acetylaspartic acid, as these compounds have been found to be absorbed particularly easily into the skin of the face, body and/or scalp. Preferably, the cosmetic or dermatological compositions of the invention may comprise, in a physiologically acceptable medium, at least one of naringenin, gibberellic acid and N-acetylaspartic acid. In some uses a composition comprising naringenin is preferrred. In other uses a composition comprising gibberellic acid is preferred. In other uses still, a composition comprising N-acetylaspartic acid is preferred.

In one preferred aspect of the invention, a compound selected from the group consisting of: gibberellic acid, naringenin, N-acetylaspartic acid, β-Aescin, arachadonic acid, quercetin, vitexin and docosahexaenoic acid (ethyl ester) may be used to inhibit matrix metalloproteinase (MMP) expression, which can be induced by UV-exposure and by inflammatory processes in the skin. Accordingly, these compounds can be used to prevent or alleviate the formation of skin wrinkles and skin laxity associated with collagen which has been lost or destroyed, while maintaining healthy skin integrity. Suitably, the MMP may be selected from at least one of, or any combination or two or more of MMP-1, MMP-3, MMP-9, MMP-2, MMP-8, and MMP-13. In particular, the compounds naringenin, gibberellic acid and N-acetylaspartic acid are preferred, as they have been shown to be potent inhibitors of these MMPs. A particularly preferred compound for use in this application is naringenin. A further particularly preferred compound for use in this application is gibberellic acid. A further still particularly preferred compound for use in this application compound is N-acetylaspartic acid.

In a related preferred aspect, the compounds of the invention, gibberellic acids, naringenins, N-acetylaspartic acids, β-aescin, arachadonic acid, quercetin, vitexin and docosahexaenoic acid (ethyl ester) have been shown to promote pro-collagen 1 expression. In particular, naringenin, gibberellic acid and N-acetylaspartic acid, DEE and BE have been found to be particularly good pro-collagen expression promoters. Suitably, at least one or combination of at least two or more of the compounds of the invention or the preferred compounds of the invention, can be used to treat and/or prevent effects of collagen reduction in the skin, that is, prevent or alleviate at least one of the formation of skin wrinkles and fine lines, reduce the severity of skin wrinkles, and prevent against skin laxity and other aging signs associated with reduced collagen levels. A particularly preferred compound for use in this application is naringenin. A further particularly preferred compound for use in this application is gibberellic acid. A further still particularly preferred compound for use in this application compound is N-acetylaspartic acid.

In a related preferred aspect, the compounds of the invention, gibberellic acids, naringenins, N-acetylaspartic acids, β-aescin, arachadonic acid, quercetin, vitexin and docosahexaenoic acid (ethyl ester) have been shown to inhibit production of the pro-inflammatory cytokine interleukin-6 in dermal fibroblasts. The compounds of the invention therefore provide an effective treatment for wrinkles which counteracts the damage caused by photo damage, but also the inflammation induced damage arising from pro-inflammatory cytokine production and enhanced MMP production arising from same, and ROS generation arising from the inflammatory process. In particular, the compounds gibberellic acid, naringenin, N-acetyl aspartic acid, have been shown to inhibit production of the pro-inflammatory cytokine interleukin-6 in dermal fibroblasts. Suitably, the compounds of the invention, and particularly, naringenin, gibberellic acid and N-acetylaspartic acid can be used to prevent against and to alleviate the symptoms of skin inflammation and related inflammatory photo-aging symptoms, such as wrinkle formation and skin darkening. A particularly preferred compound for use in this application is naringenin. A further particularly preferred compound for use in this application is gibberellic acid. A further still particularly preferred compound for use in this application compound is N-acetylaspartic acid.

In a related aspect of the invention, the compounds described herein have been found to inhibit GM-CSF and can therefore be used to prevent and/or treat unwanted skin pigmentation and/or to prevent and/or to treat inflammation-induced redness associated with aging and UV damage. Thus symptoms of elevated levels of GM-SCF which can be prevented or alleviated. A particularly preferred compound for use in this application is naringenin. A further particularly preferred compound for use in this application is gibberellic acid. A further still particularly preferred compound for use in this application compound is N-acetylaspartic acid.

In a related aspect, the compounds of the invention have been shown to inhibit VEGF production in and particular, naringenin, gibberellic acid and N-acetylaspartic acid have been shown to have inhibitory effect on VEGF. Therefore the compounds of the invention can be used to treat and or alleviate at least one of the following signs of skin aging: skin redness caused by UV irradiation or inflammatory action, puffiness around the eyes and broken veins. The compounds of the invention can therefore be advantageously used to counteract these ageing effects on the skin appearance. A particularly preferred compound for use in this application is naringenin. A further particularly preferred compound for use in this application is gibberellic acid. A further still particularly preferred compound for use in this application compound is N-acetylaspartic acid.

In a further related aspect, the compounds of the invention have been shown to stimulate fibronectin production and in particular, arachidonic acid, β-aescin and docosahexaenoic acid (ethyl ester) have been shown to stimulate higher fibronectin production than retinoic acid in comparative studies. Furthermore, the compounds of the invention, and in particular, naringenin, gibberellic acid and N-acetylaspartic acid have been shown to stimulate fibroblast growth factor production. Naringenin, gibberellic acid and N-acetylaspartic acid are particularly preferred, as they have been shown to result in higher FGF production than retinoic acid. Since, autologous fibronectin has a role in promoting healing of chronic skin and promoting re-epithelialisation and FGF is one of the growth factors involves in the wound healing process and has been showed to help restore deficiencies in impaired wound healing and it has been suggested as a treatment for patients with deficient wound repair (Greenhalgh et al). These compounds may be advantageously used improve the skin wound healing. A particularly preferred compound for use in this application is naringenin. A further particularly preferred compound for use in this application is gibberellic acid. A further still particularly preferred compound for use in this application compound is N-acetylaspartic acid.

In a further related aspect still, the compounds of the invention, arachidonic acid, quercetin, N-acetyl aspartic acid, docosahexaenoic acid (ethyl ester), gibberellic acid, naringenin and β-aescin have been shown to induce production of Kallikrein 8. In particular, naringenin, gibberellic acid and N-acetylaspartic acid have good Kallikrein 8 inducing effects. The compounds of the invention can therefore be used to combat or alleviate signs of skin aging by enhance skin desquamation, improving skin hydration, skin lustre and brightness. In a further aspect, the compounds of the invention, particularly, arachidonic acid, quercetin, N-acetyl aspartic acid, docosahexaenoic acid (ethyl ester), gibberellic acid, naringenin and β-aescin have been shown to stimulate production of Keratin 1/10. The compounds of the invention can therefore be used to maintain healthy skin condition and hydration, skin lustre and brightness. A particularly preferred compound for use in this application is naringenin. A further particularly preferred compound for use in this application is gibberellic acid. A further still particularly preferred compound for use in this application compound is N-acetylaspartic acid.

Suitably, the compositions of the invention may comprise excipients suitable for topical application to the skin, body and/or scalp. Desirably, the composition according to the invention may be in the form of conventional skin-care products. Preferably, the composition is in the form of a cream, lotion or serum. The composition may alternatively be in the form of conventional skin-care products such as a gel, cream, milk, lotion, serum, oil (for example massage oil), scrub, powder, mask, toner, or the like. The composition may also be in the form of a soap or a cleanser (such as a facial cleanser), a shampoo, a shower or bath gel. Furthermore, the composition may also be a colour cosmetic product such as a foundation, a base for make-up, a concealer, pressed powder, mascara or a lipstick. It may also be incorporated into a wrap or film, a mask, a patch, a cloth or blanket, a pad, a sheet, a wipe, a pen or the like. Most preferably the product is a leave-on topical product, that is, a product to be applied to the skin without a deliberate rinsing step soon after its application to the skin.

Suitably, the compositions of the invention may further comprise at least one of a sunscreen or UV filter(s), depigmenting agents for lightening the skin tone of a subject, UV filters, moisturizing agents or further cosmetic agents or anti-aging components.

In a preferred embodiment of the invention, the compositions of the invention comprise at least one further cosmetically active agent, selected from the group consisting of: a retinoid and/or derivatives thereof, depigmenting agents, sunscreens or UV filters, organic photoprotective agents and/or at least one inorganic photoprotective agents, peeling agents, antioxidants, moisturizing agents, etc. and mixtures thereof.

The compositions of the invention may further comprise agents selected from the group consisting of: silicones, emulsifiers, thickeners, powders, film formers, rheology modifying agents, propellants and combinations thereof. It will be appreciated by the person skilled in the art that the composition may comprise other suitable adjuncts. For example, the composition may further include adjuncts such as fragrance, opacifiers, preservatives, colorants, pigments, buffers, chelating agents, sensory enhancers, etc.

The compositions of the invention may further comprise skin-care actives such as UV filters (such as ethylhexyl methoxycinnamate, octocrylene, ethylhexyl salicylate, butyl methoxydibenzoylmethane, titanium dioxide or phenylbenzimidazol sulfonic Acid, for example), anti-ageing actives (for example vitamins or proteins), anti-wrinkle actives, moisturising actives, antioxidants, skin purifying actives, peeling agents, sebum reducing agents, mattifying agents, anti-perspirant actives, self-tanning actives, skin plumping actives, barrier function enhancing agents, surfactants and other cleansing agents, delivery enhancers and the like.

Suitably, skin lightening agents such as inhibitors of protein kinase A, inhibitors of protein kinase C-beta, inhibitors of GTP-cyclohydrolase-1, melanin concentration hormone receptor agonists, phenylalanine uptake inhibitors, ionotropic and metabotropic glutamate receptor antagonists and mixtures thereof, may also be included in the composition according to the invention.

In one embodiment, the composition further comprises one or more skin care actives selected from the group consisting of alpha-hydroxy acids, beta-hydroxy acids, polyhydroxy acids, hyaluronic acid, algae extracts, peptides, vitamin C derivatives, conjugated linoleic acid, allantoin, retinoids, resorcinol derivatives, alpha-arbutin, tannins, flavanoids, ellagic acid, gallic acid and mixtures thereof. In an embodiment wherein the composition comprising a least one retinoid, it is preferred that the composition does not comprise naringenin orquercetin.

The compositions of the invention may also comprise a vehicle which may be formulated to improve the delivery of the actives to the skin. The topical composition according to the present invention may be prepared in a mannerwell known in the art of preparing skin care products. The active components are generally incorporated in a dermatologically acceptable vehicle or carrier. The active components can suitably first be dissolved or dispersed in a portion of the water or another solvent or liquid to be incorporated in the composition.

The composition may be in the form of an emulsion, such as the oil-in-water, water-in-oil, silicone-in-water, water-in-silicone types, or a multiple emulsion such as a triple emulsion (for example water/oil/water (W/O/W) emulsion), phase inversion temperature (P.I.T.) emulsion, phase inversion concentration (P.I.C.) emulsion, wax-in-water emulsion, microemulsion or D-phase gel or the like. Compositions may also be in the form of a cream, gel, a solution, a dispersion (for example a hydro-dispersion or lipo-dispersion), a paste or a solid (for example, a solid stick, pressed powder). Alternatively, the compositions may be in the form of an alcohol-based system or an aerosol.

In a preferred embodiment, the composition is in the form of an emulsion, in particular an oil-in-water emulsion or a water-in-oil emulsion

The compounds which form the invention could be used in hydrous or anhydrous compositions, for example, formulated and/or packaged into a water-free environment [formulation or packaging or encapsulated delivery system]. Anhydrous compositions are particularly preferred. In a preferred embodiment, the compounds of the invention may be formulated to be contained within vesicular systems, e.g. phospholipid, lecithin. Solid or semi-solid shell encapsulating materials may be used to enclose the compounds of the invention or a composition comprising the compounds of the invention. Alternatively the compound or compositions comprising the compound may be encapsulated within porous organic polymer microspheres (see US5,851,538), sol-gel microcapsules (see US2010255107), or mesoporous microparticulate materials (see US2001/0236493).

In a further preferred embodiment, the compounds may be formulated in a non-solvent (see US6103267) or nonaqueous system and packaged within one chamber of a dual-chamber dispensing system, such as that described in WO9727841, in order to be mixed with a composition in the second chamber close to or at the point of application. The compound could be formulated in an essentially dry form, as a powder composition which may or may not be mixed with water (WO2011121540) or a second composition, at point of use (such a method has been described in WO201112112)

The composition may be packaged in any suitable manner such as a jar, a bottle, a tube, a pump, a pump dispenser-tube, an aerosol or foam dispensing pump, a roll-ball, a stick, a brush or a sachet, for example. It may also be incorporated in a capsule, an ampoule or a pipette (for example a dropper system). Suitably, where a retinoid is included in the composition of the invention, it may be selected from retinol, retinal, retinoic acid (e.g. tretinoin, all-trans retinoic acid), an ester of retinol and an acid, C₂-C₂₀, such as propionate, acetate, linoleate, or retinol palmitate.

In a preferred embodiment, the composition of the invention comprises a retinoid selected from retinol, retinal, retinoic acid, an ester of retinol and an acid, C₂-C₂₀, such as propionate, acetate, linoleate, or retinol palmitate. In these embodiments wherein a retinoid is present, it is preferred that the composition does not comprise naringenin or quercetin.

In a preferred embodiment UVfilters are present in the cosmetic or dermatological composition in a concentration in the range from about 0.1 % - 30% w/w, and more preferably in the range from about 0.5% -10% w/w based on the total weight of the topical cosmetic composition. In a related aspect, the composition of the invention may be provided in the form of:
(i) a care, treatment, cleansing or protective product for skin, including the face and/or scalp;
(ii) an anti-wrinkle or anti-ageing composition for the face;
(iii) a composition for irritated skin;
(iv) an anti-sun damage composition;
(v) an artificial tanning composition for the face and/or body;
(vi) an after-sun care composition; or
(vii) an anti-acne/anti-blemish composition.Suitably, the compositions described herein may be used in any one or all of the anti-aging uses described herein.

In a related aspect of the invention, there is provided a non-therapeutic cosmetic or dermatological procedure to prevent and/or delay and/or limit and/or treat at least one sign of skin ageing or the skin damage condition resulting from intrinsic or photo-induced ageing, comprising the step of applying to the skin, body and/or scalp of a subject an effective amount of the at least one compound of or at least composition of the invention

Desirably, the application of the compounds and or compositions of the invention to the skin, body and/or scalp:
(i) lightens the colour of the skin or scalp of a subject, by preventing the formation and/or removing brown pigment blemishes and/or age blemishes and/or freckles; and/or
(ii) reduces at least one of skin redness, blotchiness or inflammation; and/or
(iii) reduces and/or smoothes skin imperfections such as wrinkles and/orfine lines; and/or
(iv) produces more homogenous, firmer, more toned and more elastic skin; and/or
(v) promotes wound healing; and/or
(vi) puffy eyes and/or dark circles; and/or
(vii) protects against UV-photo induced damage or degradation of collagen; and/or
(**viii**) improves the appearance of acne and/or blemishes.

Suitably, the concentration of the at least one active compound in the cosmetic or dermatological composition of the invention may be in the range of about 0.001- 50 % w/w. Preferably, the at least one active is in the range 0.01-10 % w/w. More preferably still the at least one active is in the range 0.05- 5 % w/w. Preferred actives are gibberellic acid, naringenin, N-acetyl aspartic acid. Most preferred is N-acetyl aspartic acid.The term *"dermatologically acceptable carrier"* as used herein, means that the carriers described are suitable for use in contact with mammalian keratinous tissue without causing any adverse effects such as undue toxicity, incompatibility, instability, allergic response, for example.

### Description of the Drawings:

Additional features and advantages of the present invention are described in, and will be apparent from, the detailed description of the invention and from the drawings in which:
**Figure 1** demonstrates the profile of penetration gibberellic acid (GA), naringenin (NG) and N-acetyl aspartic acid (Triple A) after 24-hours application on pig skin sample.
**Figure 2** demonstrates the inhibition of UV-induced MMP-1, MMP-2, MMP-3, MMP-8, MMP-9 production using gibberellic acid (GA) or N-acetyl aspartic acid (Triple A)
**Figure 3** demonstrates the inhibition of PMA inflammation induced MMP1 production using naringenin (NG) or gibberellic acid (GA) or N-acetyl aspartic acid (Triple A) on human dermal fibrblasts
**Figure 4** demonstrates the inhibition of PMA inflammation induced MMP3 production using naringenin (NG) or gibberellic acid (GA) or N-acetyl aspartic acid (Triple A) on human dermal fibroblasts
**Figure 5** demonstrates procollagen-1 stimulation in dermal fibroblasts after 24 hour stimulation with compounds of the invention: gibberellic acid (GA), naringenin (NG), N-acetylaspartic acid (Triple A), β-aescin (BE), arachadonic acid (AA), quercetin (QC), and docosahexaenoic acid (ethyl ester) (DEE), and control DMSO, retinoic acid (RA). Figure shows the average of 3 separate experiments in 3 different donors.
**Figure 6** demonstrates the stimulation of fibronectin in human keratinocytes stimulated with all-trans retinoic acid (RA), retino (Rol)I, arachidonic acid (AA), β-aescin (BE), docosahexaenoic acid (ethyl ester) (DEE), and the positive control calcium (Ca2+).
**Figure 7** demonstrates the inhibition of interleukin-6 [IL-6] production in dermal fibroblasts by gibberellic acid (GA), naringenin (NG) and N-acetyl aspartic acid (Triple A) compared with all-trans retinoic acid (RA), retinol.
**Figure 8** demonstrates the inhibition of GM-CSF production in human dermal fibroblasts by gibberrellic acid (GA), naringenin (NA) and N-acetyl aspartic acid (Triple A).
**Figure 9** demonstrates the effect on topical treatment of skin explants with 1 % of gibberellic acid (GA), naringenin (NG) and N-acetyl aspartic acid (Triple A) in propylene glycol on the suppression of UV-induced expression of GM-CSF.
**Figure 10** demonstrates the inhibition of inflammation-induced VEGF by naringenin (NG), N-acetyl aspartic acid (Triple A), gibberellic acid (GA) and β-aescin (BE)compared with all-trans retinoic acid (RA), retinol.
**Figure 11** demonstrates the stimulation of keratin 6 in human keratinocytes stimulated with naringenin (NG), β-aescin (BE) and docosahexaenoic acid (ethyl ester) (DEE), retinoic acid (RA) and retinol (Rol) and the positive control calcium.
**Figure 12** demonstrates the stimulation of fibronectin in human keratinocytes stimulated with all-trans retinoic acid (RA), retinol (Rol), β-aescin (BE), arachadonic acid (AA), and docosahexaenoic acid (ethyl ester) (DEE) and the positive control calcium (Ca2+).
**Figure 13** demonstrates the stimulation of FGF production in human skin explants by naringenin (NG), gibberellic acid (GA), N-acetylaspartic acid (Triple A) and retinoic acid (RA).
**Figure 14** demonstrates the significant stimulation of kallikrein 8 by gibberellic acid (GA), naringenin (NA), N-acetyl aspartic acid (Triple A), arachidonic acid (AA) and docosahexaenoic acid (ethyl ester) (DEE), as well as positive controls retinol and retinoic acid (RA).
**Figure 15** demonstrates the synthesis of keratin 1/10 expressed at % of vehicle control synthesis on human keratinocytes with naringenin (NG), gibberellic acid (GA), N-acetyl aspartic acid (Triple A), arachidonic acid (AA), β-aescin (BE), docosahexaenoic acid (ethyl ester) (DEE), all-trans retinoic acid (RA) and retinol (Rol).

### Detailed Description of the Invention:

The invention relates to compounds having several anti-aging benefits.

The inventors have identified retinoic acid-mimicking compounds which have similar gene-regulating signatures to retinoic acid and which may be used in compositions for cosmetic use. In particular, they have identified ingredients which can help protect against and alleviate the signs of aging. They have demonstrated hitherto unknown biological activity for the identified ingredients using an array of *in vitro* tests. The *in vitro* tests explored the influence of the compounds of the invention on key biological markers in the skin which are known to have an influence on skin aging.

Moreover, and advantageously, the compounds of the invention were found to be non-irritating when applied in pure form to the skin.

### Matrix Metalloproteinase [MMPs] Inhibitors - Targets wrinkles and sagging skin through inhibition/reduction of collagen degradation

UV irradiation increases the expression of Matrix metalloproteinase enzymes in the skin (Fisher et al. 1997; Farage et al. 2008). MMPs are capable of degrading all kinds of extracellular matrix proteins (Fisher et al. 1997). The MMPs are initially synthesized as inactive enzymes with a pro-peptide domain that must be removed before the enzyme is active. Under normal conditions MMPs are involved in the remodeling of the extracellular matrix, for example, in tissue repair, but when induced in excess by UV light or via inflammatory stimulation, MMP activity causes degradation and tissue-remodeling to an extent which results in connective-tissue damage and pre-mature aging (Kahari and Saarialho-Kere 1997a; Fisher et al. 1997). Different types of MMP have different substrate preferences. Several MMPs are involved in the degradation of collagen: MMP-1 starts by cleaving collagen, which is then further degraded by MMP-3 and MMP-9. Also, MMP-2, MMP-8, and MMP-13 are involved in the degradation of collagen (Kerkvliet et al. 1999). Compounds which inhibit MMP expression will prevent or reduce MMP induced collagen loss or destruction.

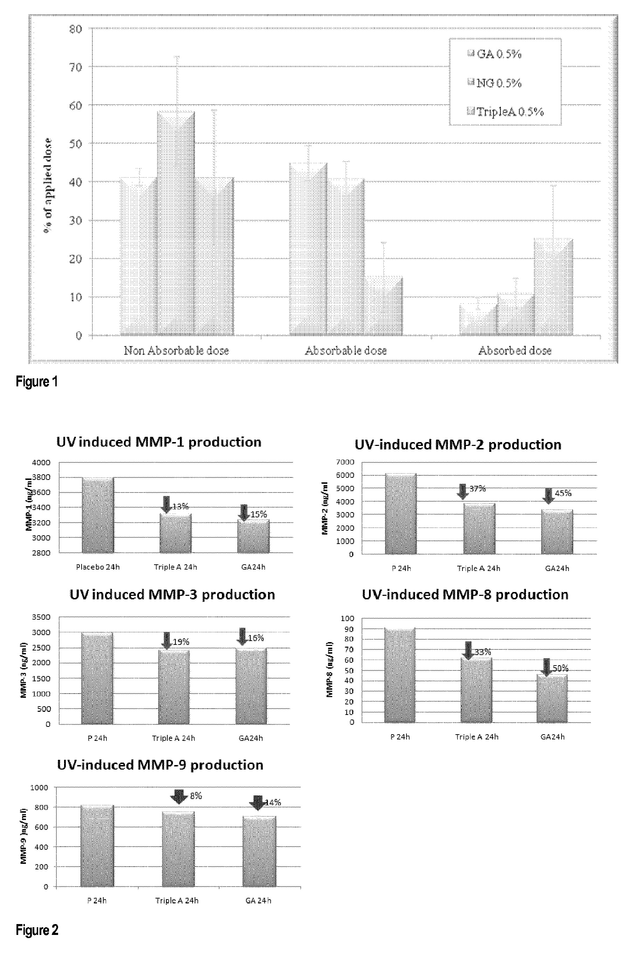

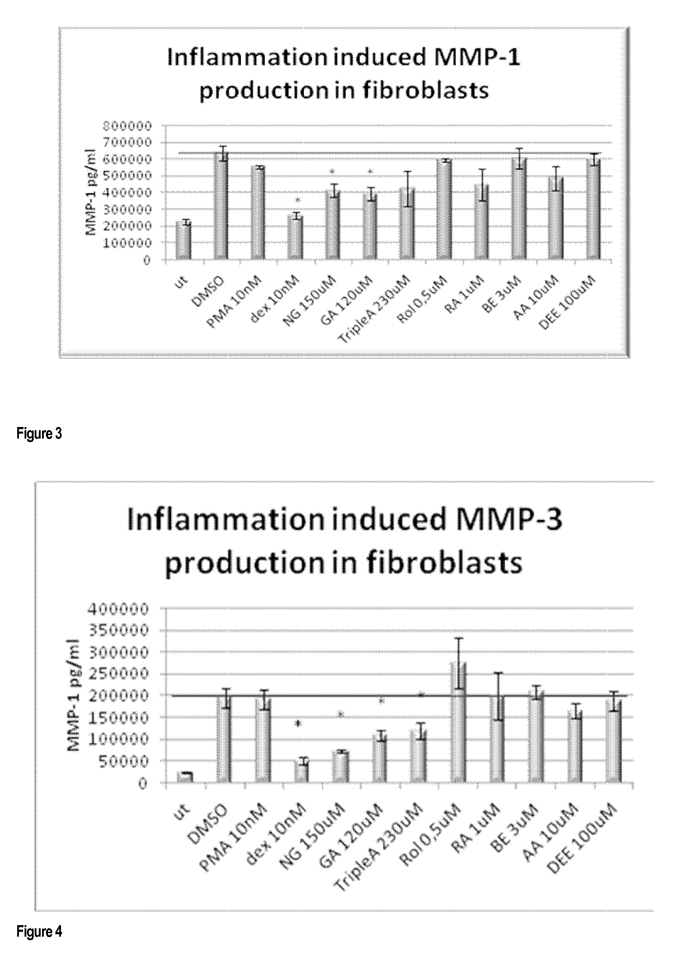

In view of the fact that the compounds of the invention have been shown to inhibit MMP expression (Figure 2 & Figure 3), the compounds can therefore be used to prevent/or alleviate the formation of skin wrinkles and skin laxity associated with collagen lost or destruction. A further advantage being that healthy skin integrity is maintained.

### Pro-collagen 1- Targets wrinkles and sagging skin through promotion of collagen synthesis

UV light not only mediates the destruction of collagen it also impairs the ongoing collagen synthesis by inhibiting the gene expression of procollagen (Kahan and Saarialho-Kere 1997b).

The compounds of the invention, gibberellic acid, naringenin, N-acetylaspartic acid, β-aescin, arachadonic acid, quercetin, vitexin and docosahexaenoic acid (ethyl ester) have been shown to promote pro-collagen 1 expression (Figure 4). In particular, naringenin, gibberellic acid and N-acetylaspartic acid, DEE and BE have been found to be particularly good pro-collagen expression promoters.

The results indicate that the compounds of the invention, can be used to prevent effects of collagen reduction in the skin, that is, prevent or alleviate the formation of skin wrinkles and fine lines, reduce the severity of skin wrinkles and prevent against skin laxity and other signs associated with reduced collagen levels.

### Pro-inflammatory cytokines - Targets Inflammation

With aging including photoaging, the skin is known to become more susceptible to inflammation. Inflammation is a defence mechanism for the skin against various forms of attack: physical, chemical, and biological. UV light induces collagen degradation through increased MMP production, and inflammation also results in an increased expression of MMP (Bennett et al. 2008; Kahari and Saarialho-Kere 1997b). Pro-inflammatory cytokines, including IL-6, are mediators inducing the expression of several MMP's, including MMP-1, MMP-2, MMP-3, MMP-9 (Dasu et al. 2003; Kossakowska et al.1999; Parks et al. 2004. IL-6 has also been implicated in the UV-induced induction of the collagen degrading MMP-1(Wlaschek et al. 1993). Interleukin -6 (IL-6) is produced at sites of inflammation (Kessler-Becker et al. 2004), and the levels of this cytokine are elevated in inflammatory skin conditions such as psoriasis (Grossman et al.1989). Expression of IL-6 has also been correlated to the epidermal hyperplasia seen in psoriatica skin. Epidermal hyperplasia is one of the features associated with photoaging skin (Rijken and Bruijnzeel 2009).

Inflammation also results in the generation of reactive oxygen species (ROS), which is involved in several destructive processes in the skin, further accelerating the aging process (Meier et al.1989) (Conner and Grisham 1996).

An effective treatment for wrinkles needs to address the damage caused by photo damage, but also the inflammation induced damage arising from pro-inflammatory cytokine production and enhanced MMP production arising from same, and ROS generation arising from the inflammatory process.

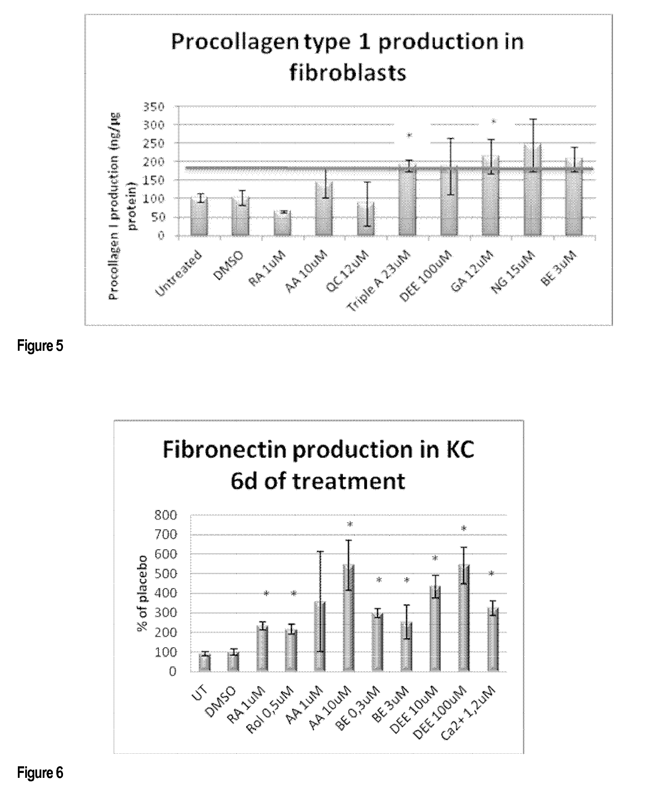

The compounds of the invention, and in particular, gibberellic acid, naringenin, N-acetyl aspartic acid, have been shown to inhibit production of the pro-inflammatory cytokine interleukin-6 (Figure 6) in dermal fibroblasts . Therefore the compounds of the invention, and particularly, naringenin, gibberellic acid and N-acetylaspartic acid can be used to prevent against and alleviate the symptoms of inflammation and related inflammatory photo-aging symptoms, such as wrinkle formation and skin darkening.

### GM-CSF- Targets skin depigmentation and prevents against inflammation induced redness

Skin pigmentation is due to the presence of melanin, (a pigment produced in the epidermis by the hydroxylation of tyrosine by the enzyme tyrosinase). Melanin pigments are made in specialized cells called melanocytes, packed in organelles called melanosomes that are transferred to keratinocytes to assure proper diffusion throughout the epidermis. The terms "whitening/pigmentation agents" refer to active agents that are able to reduce or modulate skin pigmentation by limiting melanin production, inhibiting melanosome maturation and transfer, or by stimulating pigment degradation.

Skin lightening products are commercially available for cosmetic purposes in order to obtain lighter skin complexion. Clinically they are also used for treatment of hyperpigmentary disorders such as melasma, café au lait spot and solar lentigo. All of these target naturally melanin production and many of the commonly used agents are known as competitive inhibitors of tyrosinase, one of the key enzymes in melanogenesis (Hearing and Jimenez 1987;).

However, melanogenesis is not only controlled by tyrosinase, instead there are several factors that play an important role for darkening the skin (Gillbro and Olsson 2011) such as the transfer of melanosomes from melanocytes to the surrounding keratinocytes or paracrine production of melanogeneic factors from keratinocytes. (Thody and Graham 1998) (LERNER and MCGUIRE 1961; LERNER et al. 1954; LERNER and MCGUIRE 1964) (Schaueretal. 1994). (Jabbour 2003; Levine et al. 1991) (Abe et al. 1969; Busca and Ballotti 2000; Hunt et al. 1994; Im et al. 1998).

One important mechanism seems to be paracrine production of granulocyte-macrophage colony-stimulating factor (GM-CSF). Keratinocytes responds to exposure of UVA and UVB with increased production of GM-CSF (Imokawa et al. 1996; Hirobe et al. 2004). Melanocytes possess specific binding sites for GM-CSF, and once bound GM-CSF increases pigment production in melanocytes (Imokawa et al. 1996). Even thus UVA and UVB activate keratinocytes differently both UVA and UVB results in the production of GM-CSF. Thus GM-CSF is important for the pigmentation of the epidermis and can be seen as a biomarker for skin pigmentation.

GM-CSF has been shown to play an important role in inflammation and inhibition of GM-SCF has been shown to decrease skin inflammation (Hamilton 2008). It has also been suggested that GM-CSF plays a part in sustaining inflammatory response, by inhibiting the apoptosis of monocytes, thereby contributing to the transition from acute to chronic inflammatory skin disease (Bratton et al. 1995).

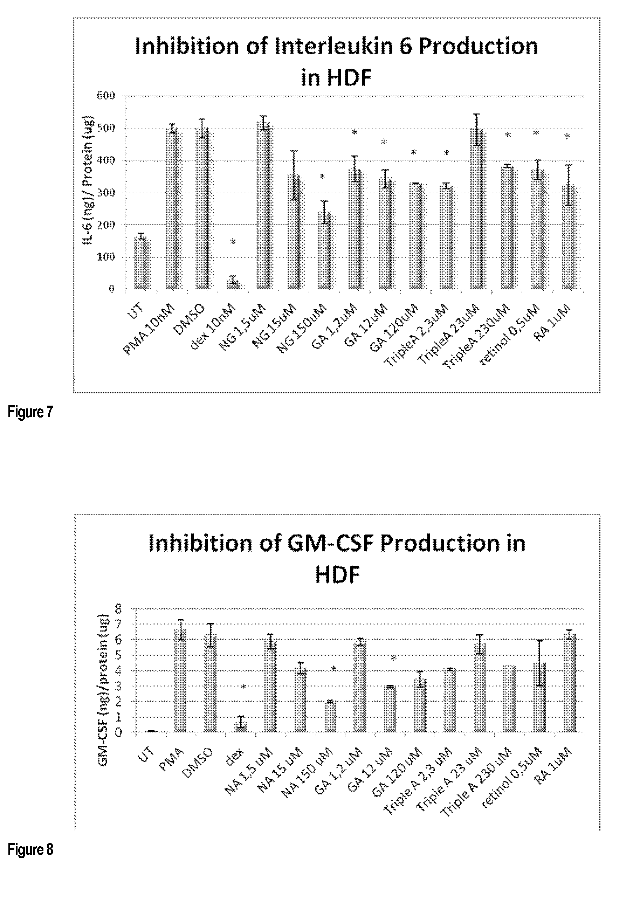

The compounds of the invention have been shown to inhibit GM-CSF (Figure 7 & Figure 8) and in and particular, naringenin, gibberellic acid and N-acetylaspartic acid have been shown to have inhibitory action on GM-CSF. The compound of the invention can therefore be used to prevent unwanted skin pigmentation and/or to prevent inflammation-induced redness associated with elevated levels of GM-SCF which can arise from aging and UV damage.

### Vascular endothelial growth factor- Targets redness, puffy eyes and broken veins

Photo damage and sunburn is a major cause of extrinsic aging. Excessive UVB exposure is accompanied by erythema (redness), edema (puffiness), vasodilatation and angiogenesis as a result of increased vascular permeability. Vascular endothelial growth factor (VEGF) is the main mediator of these changes (Hirakawa et al. 2005). Also moderate levels of UVB light induce the expression of VEFG (Brauchle et al. 1996a). The expression of VEGF is also induced by inflammation (Salven et al. 2001). VEGF is induced by UV irradiation in both skin fibroblasts and keratinocytes (Trompezinski et al. 2001) whereas inflammation-induced VEGF is mainly expressed in dermal fibroblasts. Over expression of VEGF from UV-irradiated skin or inflammatory activated skin may contribute to dilated microvasculature, a common feature of aging skin.

Edema is the cause behind puffiness and results from the increased vascular permeability induced by VEGF (Phelps 1990; Infanger et al. 2004).

Rosacea is a common skin condition characterized by vascular abnormalities in the skin, leading to erythema and telangiectasis (broken capillaries). Since rosacea is a very visible condition the impact on quality of life for affected persons are large. Receptors for VEGF are typically expressed in rosacea skin (Smith et al. 2007). Previous research has showed reduction in the size and number of vessels in an experimental model of psoriasis after VEGF blockade (Schonthaler et al. 2009).

Free radical is another threat against younger healthy looking skin. H₂O₂ is a strongly oxidizing compound and leads the formation of free radicals. It has been shown that VEGF is induced also by H₂O₂ (Brauchle et al. 1996b). Mediators produced during inflammation, such as IL-6, result in the occurrence of free radicals and H₂O₂ (Winrow et al. 1993), as well as VEGF (Cohen et al. 1996).

Reducing the amount of VEGF, both from UV irradiation and inflammation, is hence a way to combat redness and puffiness and superficial veins.

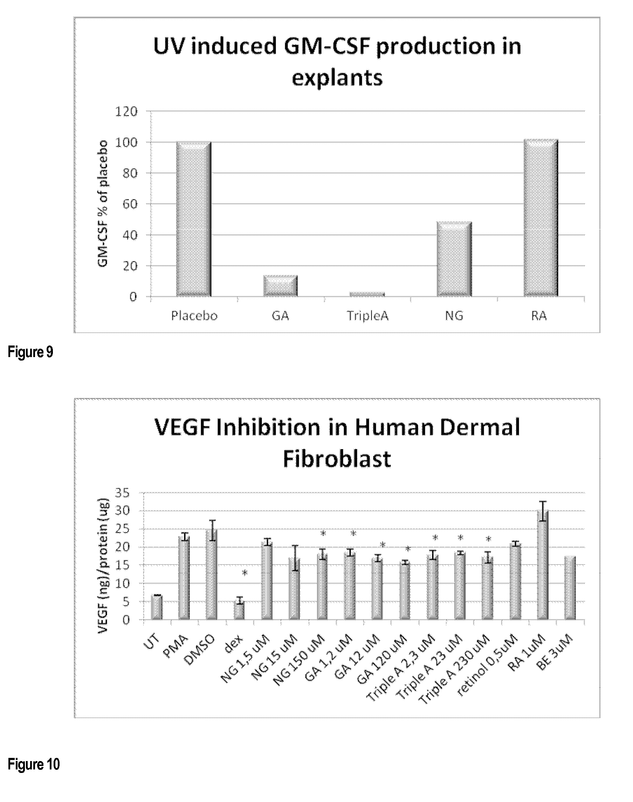

The compounds of the invention have been shown to inhibit VEGF production (Figure 9) in and particular, naringenin, gibberellic acid and N-acetylaspartic acid have been shown to have inhibitory effect on VEGF. Therefore the compounds of the invention can be used to combat skin redness caused by UV irradiation or inflammatory action, puffiness around the eyes and broken veins, all of which have an ageing effect on the skin appearance.

### Epidermal growth factor- Targets improved wound healing

Members of the epidermal growth factor (EGF) family are important growth factors involved in epithelialization during cutaneous wound healing. Heparin-binding EGF-like growth factor (HB-EGF), a member of the EGF family, has been shown to play an important role in skin wound healing where it functions by accelerating keratinocyte migration, rather than proliferation (Shirakata et al. 2005).

The compounds of the invention have been shown (see Table 3) to stimulate heparin-binding epidermal growth factor production (on a gene level) and therefore can be use can improve the skin wound healing process. Table 3 demonstrates gene data for HBEGF stimulation by naringenin when topically applied to skin explants, demonstrating skin delivery from topical application on the skin and resulting gene regulation.

### Connective tissue growth factor- Targets wound healing

Connective tissue growth factor (CTGF) is an ECM-associated heparin-binding protein that binds directly to integrins. It is synthesized by fibroblasts and stimulates proliferation and chemotaxis of these cells. It has also been demonstrated that CTGF is required for reepithelialization in wound healing by promoting cell migration (Igarashi et al. 1993). In addition, CTGF is a strong inducer of ECM proteins, such as collagen type I and fibronectin and their integrin receptors ((Secker et al. 2008).

The compounds of the invention have been shown (see Table 3) to stimulate connective tissue growth factor production (on a gene level) and thus can be used to improve and/or enhance the skin wound healing process. Table 3 demonstrates gene data for CTGF stimulation by the compounds of the invention when topically applied to skin explants, demonstrating skin delivery from topical application on the skin and resulting in gene regulation.

### Fibronectin - Targets wound healing

Autologous fibronectin has a role in promoting healing of chronic skin and corneal ulcers. Fibronectin assists in wound healing by contributing to haemostasis, assisting in control of infection and debridement of wounds, and promoting re-epithelialisation, granulation tissue and ultimately a connective tissue of adequate tensile strength to repair the skin defect.

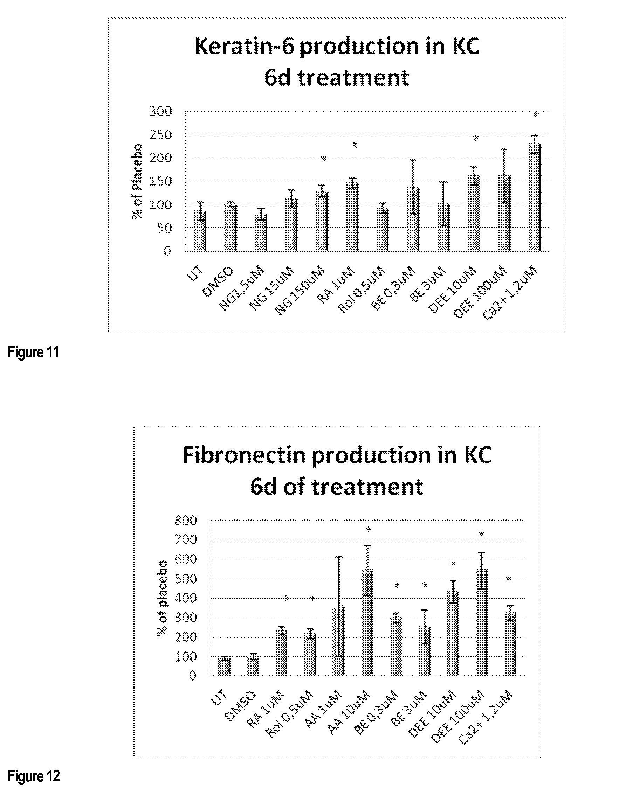

The compounds of the invention have been shown to stimulate fibronectin production and particular, arachidonic acid, β-aescin and docosahexaenoic acid (ethyl ester) (Figure 11) have been shown to stimulate higher fibronectin production than retinoic acid. The compounds of the invention can therefore be used to improve the skin wound healing process.

### Fibroblast growth factor- Targets wound healing

Fibroblast growth factor (FGF) is one of the growth factors involves in the wound healing process and has been showed to help restore deficiencies in impaired wound healing and it has been suggested as a treatment for patients with deficient wound repair. Also Plasminogen activator (PLAT) is activated by all-trans retinoic acid, Triple A and GA. The activation of the plasminogen activator system may be one mechanism by which all-trans retinoic acid exerts beneficial effects in cutaneous wound healing. The compounds of the invention, and in particular, naringenin, gibberellic acid and N-acetylaspartic acid have been shown to stimulate fibroblast growth factor production (Figure 12). Naringenin, gibberellic acid and N-acetylaspartic acid have been shown to result in higher FGF production than retinoic acid. Therefore these compounds used improve the skin wound healing process.

### Kallikrein 8 - Targets desquamation, skin hydration, skin lustre and brightness

Skin renewal begins with the generation of new keratinocytes from stem cells residing in the stratum basale, the inner layer of the epidermis. As new cells keep forming, keratinocytes migrate upward and differentiate into comeocytes. Keratin, which is a highly fibrous protein, is produced during the differentiation process and causes cell walls to harden, forming the stratum corneum (SC). The terminal differentiation of keratinocytes ultimately results in cell death. Old corneocytes are shed from the SC through desquamation. Aging is associated with reduced epidermal proliferation.

Kallikreins are serine proteases and thought to be responsible for the proteolysis of desmosomes during desquamation (Komatsu et al. 2005). Kallikrein 8 (KLK8) is involved both in epidermal proliferation and comeocyte shedding (Kishibe et al. 2007) both directly, and through the induction of KLK 6 and KLK 7.

Although RA is very effective stimulating the desquamation process by inducing high production of Kallikrein it also known to be somewhat pro-inflammatory which is activating other processes involved in the aging of the skin negatively.

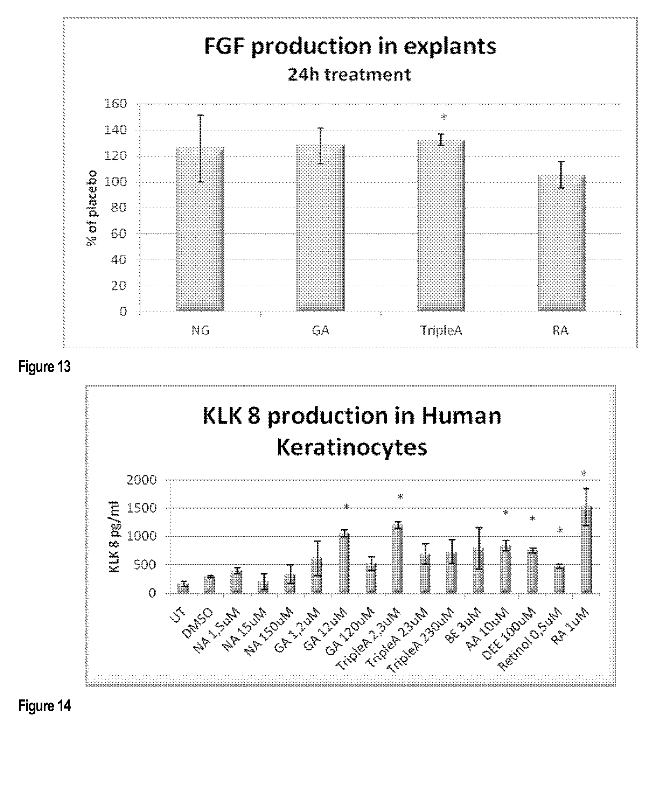

The compounds of the invention, arachidonic acid, quercetin, N-acetyl aspartic acid, docosahexaenoic acid (ethyl ester), gibberellic acid, naringenin and β-aescin have been shown to induce production of Kallikrein 8 (Figure 13). In particular, naringenin, gibberellic acid and N-acetylaspartic acid have good inducing effects. The compounds of the invention can therefore be used to enhance skin desquamation, improve skin hydration, skin lustre and brightness.

### Keratin 1/10- Targets epidermal differentiation, hydration, and healthy looking skin

Skin is composed of two layer, the outer epidermis and the inner dermis (Proksch et al. 2008) Keratinocytes compose the outermost layer of the epidermis, the stratum corneum, which provides a physical and chemical barrier towards the environment, preventing the entry of foreign substances and the loss of water and important constituents of the skin and body (Borgono et al. 2007). The keratinocytes in the inner layer of the epidermis, the basal layer, are proliferating and undifferentiated. When they start to differentiate they exit the cell cycle and start moving upward in the epidermis towards the surface of the skin (Fuchs 2007). The keratinocytes are linked to each other by cohesive mechanical junctions named desmosomes (Sandjeu and Haftek 2009). Keratin 1 and keratin 10 are early markers of keratinocyte differentiation. Keratin 1 and Keratin 10 work as a dimer (Keratin1/10) and are expressed together. As the differentiation process continues the expression of keratin 1 and keratin 10 is downregulated again and is replaced by other molecules i.e. involucrin and filaggrin (Lu et al.1994). There is evidence suggesting that increased levels of keratin 1/10 are associated with reduced tumongenesis (Tiano et al. 2002).]

With differentiation, keratinocytes move upward and became granular keratinocytes (GKs). GKs produce almost all of the proteins and lipids required for the protective barrier function undergoing a specially programmed cell death called cornification (Toulza et al. 2007). The cornification process gives rise to comeocytes, they are devoid of cell organelles and mainly consist of keratin. Eventually the corneocytes at the outermost layer of the stratum corneum are gradually shed and replaced by newly-keratinised cells in a process called desquamation (Hara et al. 2011). To maintain homeostasis and a functional barrier the delicate balance between proliferating and differentiating keratinocytes need to be tightly regulated.

The compounds of the invention, arachidonic acid, quercetin, N-acetyl aspartic acid, docosahexaenoic acid (ethyl ester), gibberellic acid, naringenin and β-aescin have been shown have been shown to stimulate production of Keratin 1/10. The compounds of the invention can therefore be used to maintain healthy skin condition and hydration, skin lustre and brightness.

### EXAMPLES

### Example 1 - Connectivity mapping approach identifies compounds with retinoic acid-like gene regulation behaviour

A connectivity mapping (Cmap) approach was used to identify retinoid-like compounds.

Gene expression profiling was performed on skin biopsies from healthy female subjects which were topically applied with tretinoin (all-trans retinoic acid) in a concentration of 0.025% (Retin-A® ex. Johnson & Johnson) for one week. Gene signatures obtained from the tretinoin treated skin were queried in the Cmap database. Compounds identified which ranked highly in the Cmap were topically applied on skin explants received from breast plastic surgery. Gene arrays were repeated on the treated samples 24 hours after topical application.

### Example 1.1 Materials and methods

### Treatment of skin with tretinoin

Skin biopsies were taken from sun-exposed forearms of 8 healthy Causcasion female subjects, aged 50+ years. Patients were treated with tretinoin (Retin-A® concentration 0.025%) for one week. At the end of the week 3mm punch biopsies were taken from each site of treatment. The study was vehicle controlled. Two biopsies were taken per treatment, per arm, for sufficient RNA concentration. Biopsies were immediately stored under controlled conditions at 4°C for 24 hours prior to RNA extraction.

### Treatment of skin explants with tretinoin

Subcutaneous fat from full thickness skin, obtained from surgical waste material from breast reduction procedures, was removed with a scalpel. Three healthy Causcasian female donors were used for this study, aged 23,41 and 60 years. The female donors differed from those of the skin-treatment study outlined previously. 8mm punch biopsies were taken from the skin (and these biopsies are now referred to as skin explants). Skin explants were put on Milipore cell culture inserts (12mm diameter). Inserts containing skin explants were put in 6-well plates (one insert /well) and 1 mL of supplemented keratinocyte medium (ex Cascade Biologics) was added to each well.

A tretinoin-containing cream (Aberela ® ex. Janssen): 0.05% tretinoin in the cream, was applied topically to each explant (5µL/cm²) using a positive displacement pipette. A placebo cream without tretinoin was used as a control. The skin explants were incubated with tretinoin for 24 hours at 37 °C in 5% CO₂-humidified air.

Viability of explants was controlled using parallel Alamar blue test using suppliers protocol (ex. Invitrogen)

### RNA extraction of human biopsies

A hand-held Qiagen Tissue Ruptor mini kit was used for RMA extraction from skin biopsies as per the supplier protocol. Qiagen Tissue Ruptor was used to disrupt the tissue in Qiazol (ex. Qiagen) according to the supplier protocol. Qiagen minikit was used for RNA extraction according to the manufacturer's protocol. RNA was stored in -80°C prior to cDNA conversion.

### Microarray expression analysis

RNA concentration was measured with ND-1000 spectrophotometer (ex. NanoDrop Technologies) and RNA quality was evaluated using the Agilent 2100 bioanalyzer system (ex. Agilent Technologies).

250 nanograms of total RNA from each skin or skin explant sample was used to prepare biotinylated fragmented cRNA according to the GeneChip® 3'IVT Express Kit Manual (PN702646 Rev 1, ex. Affymetrix Inc.). Affymetrix GeneChip® expression arrays (Human Genome U133 Plus 2.0) were hybridized for 16 hours in a 45°C incubator, rotated at 60 rpm. According to the GeneChip Expression Wash, Stain and Scan Manual (PN 702731 Rev 2, ex. Affymetrix Inc.) the arrays were washed and stained using the Fluidics Station 450 and scanned using the GeneChip® Scanner 3000 7G.

### Microarray data analysis

Analysis of the gene expression data was carried out in the statistical computing language R (http://www.r-project.org) using packages available from the Bioconductor project (www.bioconductor.org). The raw data was normalized using the robust multi-array average (RMA) method. In order to search for differentially expressed genes between the X samples and the Y samples group an empirical Bayes moderated t-testwas then applied using the 'limma' package. *Example 1.2 Results-Identification of*

### compounds with gene signatures mimicking retinoic acid

The findings from the Cmap analysis connected the gene signature of retinoic acid-treated human skin to novel potential compounds with the ability to mimic the activity of retinoic acid.

Many genes were individually shown to be regulated by all-trans retinoic acid. Due to the intervariable differences in the patient material from the explants study and clinical study, separate approaches were taken. The gene expression profiles in biopsies after all-trans retinoic acid treatment compared with vehicle-treated controls were examined by DNA microarray analysis.

Two separate studies on gene expression profiles in human skin after topical application of all-trans retinoic acid were conducted. The first study was performed Ex vivo by topically applying all-trans retinoic acid for 24 hours on skin explants from 3 donors prior microarray conduction. The 2^{nd} study was a clinical In Vivo study, topically applying all-trans retinoic acid for 1 week on 8 healthy volunteers.

The genes that were upregulated more than 2 fold in the explants study and 1,4-2 fold in the clinical study were queried in Cmap. To find structures that would putatively be able to mimic the activity of retinoic acid on the human skin, the gene signature from retinoic acid that was obtained by the previously explained explant study was queried in the Cmap database. The details of the Cmap database have been described by. *Lamb et al. 2006).* In this method, the similarity between each researcher's microarray results (the query signature) and 453 microarray results for 1309 compounds (reference signatures) in the Cmap database was evaluated using the Kolmogorov- Smimov statistic, a nonparametric, rank-based pattern-matching strategy. Each reference signature in the database was scored according to its similarity with the query signature, and the extent of the similarity is described as the "connectivity score." The connectivity score ranges from _1 to _1; nearer to _1 means higher similarity, 0 means no similarity, and nearer to _1 means opposite similarity. For the query, the probe ID defined by the Affymetrix GeneChip Human Genome U133A array (Affymetrix, Santa Clara, CA, USA) was used. Here, the probe ID of U 133A corresponding to the signatures of 24 hours in explants and 1 week stimulation in an in vivo clinical study queried in the Cmap database *via* the Internet (http://www.broad.mit.edu/cmap). "Permutated results" results," which consist of the arithmetic means of the connectivity scores and the statistical significance of the replicates (calculated as "enrichment" and "permutation *P*value" at the Cmap), were used to evaluate the significance of the scores. The

The top 100 high ranked compounds that resulted for the query of the two studies described earlier (explants study and clinical study) were further analyzed following distinct selection criteria i.e. an enrichment value >0.495. The resulting suggestions of structures were searched for compounds of natural origin. Agents with reported drug use such as Atropin, Digoxigenin or Oleandomycin, substances with known toxicity such as Solanin or Tomatidin as well as compounds derived from animal origin were dismissed. Resulting from this virtual exercise a total of 8 agents was selected to be taken into further investigation as potential retinoic acid like acting ingredients (Table 1).

Importantly, in both study results all-trans retinoic acid (tretinoin) appeared as suggested compound with very high ranking (Cmap ranking number 4 in the explants study, and Cmap ranking number 1 in the clinical study) which gives a confirmation for the suitability of the model as a tool to find active agents with desired gene signature related activity.

| **Compound** | **Code** | **Cmap rank** | **Enrichment value** | **p-Value** | **Molecular weight** | **Log p** |
|---|---|---|---|---|---|---|
| Beta Aescin | BE | 20 | 0.697 | 0.00207 | 1131 | |
| Naringenin | NG | 20 | 0.673 | 0.02499 | 272 | 1.9 |
| Docosahexaenoic acid (ethyl ester) | DEE | 43 | 0,895 | 0,02312 | 356 | |
| N-Acetyl aspartic acid | Triple A | 47 | 0,673 | 0,02499 | 175 | |
| Gibberellic acid | GA | 52 | 0.603 | 0.06477 | 346 | 0.24 |
| Arachdonic acid | AA | 61 | 0.775 | 0.02285 | 304 | 6.98 |
| Quercetin | OC | 73 | 0.550 | 0.03152 | 302 | 1.48 |
| Vitexin | VX | 100 | 0.495 | 0.19057 | 432 | 1.5 |

Table 1: Table shows the list of natural compounds that were detected in Cmap and ordered depending on Cmap rank. The code describes the codes used in result graphs.

### Example 2 In vitro skin permeation by gibberellic acid, naringenin and N-acetyl aspartic acid

### Example 2.1 Method

Gibberellic acid, naringenin and N-acetyl aspartic acid were formulated at 0.5% w/w in a basic formulation as described in the Table 2 below:

| **Tradename** | **INCI** | **%w/w** |
|---|---|---|
| Glycerin | glycerin | 6.0 |
| 1,3-butylene glycol | butylene glycol | 4.0 |
| Keltrol CG ECO | xanthan gum | 0.1 |
| Myritol 318 ECO | caprylyl/capryl triglycerides | 0.5 |
| Viscolatum AT 100P | aqua, sodium polyacryoyldimethyl taurate, hydrogenated polydecene, trideceth-10 | 10.0 |
| N/A | Gibberellic acid, naringenin or N-acetylaspartic acid | 0.5 |
| Nipanox BHT | BHT | 0.1 |
| Phenonip XB | phenoxyethanol, methylparaben, ethylparaben, propylparaben | 0.7 |
| Deionised water | aqua | q.s.100 |

The *in vitro* skin permeation experiments were developed and validated according to the Organization for Economic CoOperation and Development adopted guideline 428 (OECD guideline for the testing of chemicals. Skin absorption: in vitro method. Guideline 428 (Paris, April 2004, updated January 2011)).

For this investigation, static Franz glass diffusion cells (Permegear, USA) were used. These cells consist of donor and receptor chambers in between which a piece of porcine skin was positioned (thickness 800 µm). About 20 mg/cm² of each formulation were applied on the skin surface through the donor compartment in triplicate for each formulation. The pig skin samples were equilibrated for 30 minutes, and air bubbles were removed. The cells were kept at constant temperature with a circulating water flow at 37°C and the receptor compartment content (ethanol/water 50:50) was continuously agitated by magnetic stirrers.

After an exposure time of 24 hours, the diffusion cells were dismounted and each active was analysed in each compartment.
- **the non absorbable dose:** the skin surface was washed with 10.0 ml of methanol and one tape-strip was made in order to remove the residual donor samples.
- **the potentially absorbable dose:** the tape stripping procedure was used in order to separate the *stratum comeum* from the skin sample. For this purpose, the SC of the treated area was removed by 14 successive tape-stripping using D-Squam™ (ϕ=14 mm, ex. CuDerm) with a constant pressure (225 g.cm²) for 5 s. The strips were placed in amber vial with 4 ml methanol and shaken overnight.
- **the absorbable dose:** after eliminating SC from skin samples, the skin was chopped into small pieces and the test substances were extracted with 2 ml of methanol for 24-hours under constant shaking. The receptor fluid samples were diluted before the analysis.

The day after the extraction process, all the samples were filtered through 0.22µm membrane filter into HPLC vials. The samples with gibberellic acid and naringenin, together with known concentration standards were assayed by HPLC-UV (Agilent, USA). The samples with N-acetyl aspartic acid and known concentration standards were assayed by LC/electrospray ionization-mass spectrometry (LC/ESI-MS) (Shimadzu LC-10AD, ThermoScientific).

### Example 2.2 Results

The results (Figure 1) showed that topical application of gibberellic acid, naringenin and N-acetyl aspartic acid 0.5% w/w in a basic formulation for 24 hours, resulted in the penetration of each active into the skin. Figure 1 shows the range of each ingredient in the non absorbed dose (skin surface + 1^{st} strip), the potentially absorbable dose (SC acting as a reservoir) and in the absorbed dose (quantity in the skin and in the receptor fluid).

### Example 3 MMP-inhibitory activity of gibberellic acid and N-acetyl aspartic acid (ANTI-WRINKLE, ANTI-SAGGING)

### Example 3.1 Methods

Treatment of human skin explants with compounds of the invention

MMP inhibition activity for compound of the invention was tested on human skin explants. Skin explants were sourced from 2healthy caucasian female breast skin donor of 46 and 33 years of age. A cream containing all-trans retinoic acid (tretinoin) (0,05%) was added topically using positive displacement pipette (5 ul/cm²) to each explants. A similar cream base without tretinoin was used as placebo control. N-acetyl aspartic acid and gibberellic acid were prepared in propylene glycol (1 %). Solutions were kept under nitrogen and used immediately after preparation. Propylene glycol was used as vehicle control for the compounds of the invention in this study.

### Cell culture treatment

MMP-inhibitory activity for compounds of the invention was also assessed on human dermal fibroblasts. Dermal fibroblasts were cultured in *Dulbecco's Modified Eagle Medium* (DMEM) (Invitrogen) with 10% FBS (ex. Invitrogen) and 1 % Pencillin/Streptavidin (ex. Invitrogen). To induce inflammation in the cells, PMA (phorbol myristate acetate) (ex. Sigma) was used at a concentration of 10nM. Cells were pre-treated with gibberellic acid (GA), naringenin (NG) or N-acetyl aspartic acid (Triple A) or benchmark controls (retinol and retinoic acid (RA)) or a positive control for inhibition of inflammation: dexamethasone (dex) (ex. Sigma) for 1 h before PMA was added to the culture. The cells were stimulated for 24h and the culture supernatant was collected and stored at -20°C until further analysis.

Fluorokine MultiAnalyte Profiling (xMAP) using the BioPlex (ex. Biorad) dual laser flow-based sorting and detection analyzers manufactured by Luminex corporation (Luminex, Austin, TX) was employed to measure levels of MMP in conditioned media samples of stimulated dermal fibroblasts. The technology incorporated polystyrene microspheres dyed internally with differing ratios of two spectrally distinct fluorophores to create a family of different spectrally addressed bead sets. The bead utilized in this study was conjugated with a biotinylated capture antibody specific for a unique MMPs, The assay was performed without antecedent serum depletion. Results were compared with medium from cells treated with vehicle only (DMSO). The assays utilized a 96-well microplate format and were processed according to the manufacturer's protocol, including generation of a standard curve for each target prepared in background assay diluents. Values that fell within the linear range of the calibration assay were accepted. Samples were analyzed using the Bio-Plex suspension array system and Bio-Plex Manager software 4.0 (Bio-Rad Laboratories, Hercules, CA). Quantities were determined by comparison to standard curves obtained for the different

MMP.

### Example 3.2 Results

Naringenin, gibberellic acid and N-acetyl aspartic acid show an ability to inhibit MMP production which results in less collagen breakdown in the skin (Figures 2, 3 and 4). Collagen loss contributes to unwanted features of skin aging and photo-induced aging, and through inhibition of MMP production the compounds of the invention will alleviate skin wrinkle and line formation and prevent the breakdown of skin integrity proteins which result in reduced skin elasticity and flaccidness.

### Example 4 Procollagen-1 induction activity of arachidonic acid, quercetin, N-acetyl aspartic acid, docosahexaenoic acid (ethyl ester), gibberellic acid, naringenin and β-aescin (ANTI-WRINKLE, ANTI-PHOTOAGING)

### Example 4.1 Method

Fibroblast cells were cultured as outlined in Example 3.1 and treated with compounds of the invention

The secretion of pro-collagen in the cell culture supernatants were analyzed using Pro-Collagen 1 specific ELISA (#8003; ex. Quidel). Results were compared with cell culture supernatants from DMSO vehicle treated cells. The assays utilized a 96-well microplate format and were processed according to the manufacturer's protocol, including generation of a standard curve prepared in background assay diluents. Absorbance of the assay dye was read at a Synergy HT plate reader. Values that fell within the linear range of the calibration assay were accepted. Quantities were determined by comparison to standard curve obtained for pro-collagen 1.

### Example 4.2 Results

Figure 5 shows procollagen-1 stimulation in dermal fibroblasts after 24 hour stimulation with compounds of the invention. Figure 5 shows the average of three separate experiments for three different donors.

N-acetyl aspartic acid and gibberellic acid significantly increase the production of pro-collagen type 1, the precursor to collagen type 1. Docosahexaenoic acid (ethyl ester), naringenin and β-aescin display a convincing trend towards increasing the production of pro-collagen type 1.

### Example 5 Positive stimulatory effect on fibronectin in human epidermal keratinocytes, arachidonic acid, β-aescin and docosahexaenoic acid (ethyl ester), (ANTI-WRINKLE)

### Example 5.1 Method

Keratinocytes were cultured in commercially available keratinocyte medium M154 (Cascade Biologics/Invitrogen) # M154500 with a final calcium 0.05uM. Supplements were added meeting following final concentrations of growthfactors; bovine pituitary extract (BPE), 0.2% v/v • bovine insulin, 5 µg/ml • hydrocortisone, 0.18 µg/ml • bovine transferrin, 5 µg/ml • human epidermal growth factor, 0.2 ng/ml.

Medium was changed every other day. Cells were grown to 80% confluence prior stimulation with compounds. Experiments were performed in 24-well plates. The cells were treated with individual compounds of the invention for 6 days. After 3 days the medium was refreshed. A source of calcium ions was used as a positive control. After 6 days tissue culture medium was stored in -20°C until analysis.

Experimental method as outlined in Example 3.1 was modified in order to analyze fibronectin levels extracted from keratinocytes stimulated for 6 days with all-trans retinoic acid, retinol, arachidonic acid, β-aescin, and dodecahexaenoic acid (ethyl ester) at a range of concentrations. Experiments were performed in duplicates. *Example* 5.2 *Results*

**Figure 6** **shows** stimulation of fibronectin in human keratinocytes stimulated with all-trans retinoic acid, retinol, arachidonic acid, β-aescin, docosahexaenoic acid (ethyl ester), and the positive calcium control. The compounds of the invention had superior effect on fibronectin release in human keratinocytes after 6 days stimulation compared to all-trans retinoic acid and retinol. Retinoic acid is a known activator of fibronectin in skin (Schwartz and Kligman 1995a). Decreased fibroblast contractile activity and reduced fibronectin expression are involved in skin photo-aging (Knott et al. 2010).

### Example 6 Interleukin inhibitory activity of gibberellic acid, naringenin and N-acetyl aspartic acid (ANTI-INFLAMMATION, ANTI-REDNESS, ANTI SPIDER VEINS, COMBA TS PUFFINESS AROUND EYES.)

### Example 6.1 Methods

Human dermal fibroblast cell cultures were prepared according to Example 3.1 .To induce inflammation in the cells PMA (phorbol myristate acetate) (ex. Sigma) was used at a concentration of 10nM. Cells were pre-treated with gibberellic acid (GA), naringenin (NG) or N-acetyl aspartic acid (Triple A) or benchmark controls (retinol and retinoic acid (RA)) or a positive control for inhibition of inflammation: dexamethasone (dex) (ex. Sigma) for 1 h before PMA was added to the culture. The cells were stimulated for 24h and the culture supernatant was collected and stored at-20°C until further analysis Experimental method as outlined in Example 3.1 was modified in order to analyze IL-6 levels extracted from dermal fibroblasts stimulated for 6 days with all-trans retinoic acid, retinol, arachidonic acid, β-aescin, and dodecahexaenoic acid (ethyl ester) at a range of concentrations. Experiments were performed in duplicates. *Example 6.2 Results*

Figure 7 shows inhibition of interleukin-6 [IL-6] production in dermal fibroblasts by gibberellic acid, naringenin and N-acetyl aspartic acid.

Gibberellic acid, naringenin and N-acetyl aspartic acid inhibit interleukin-6 production and therefore demonstrate an anti-inflammatory effect. The prevention of inflammation can help combat skin redness. Anti-inflammatory action can have a positive effect on the reduction ofVEGF-induced anti-aging signs, such as spider veins and puffiness around eye region.

### Example 7 GM-CSF inhibition (SKIN LIGHTENING ACTIVlTY OF GIBBERELLIC ACID, NARINGENIN AND n-ACETYLASPARTIC ACID, PREVENTION OF INFLAMMATION-INDUCED REDNESS)

### Example 7:1 Methods

Cell cultures were prepared according to Example 3.1

To induce inflammation in the cells PMA (phorbol myristate acetate) (ex. Sigma) was used at a concentration of 10nM. Cells were pre-treated with gibberellic acid (GA), naringenin (NG) or N-acetyl aspartic acid (Triple A) or benchmark controls (retinol and retinoic acid (RA)) or a positive control for inhibition of inflammation: dexamethasone (dex) (ex. Sigma) for 1 h before PMA was added to the culture. Thecells were stimulated for 24h and the culture supernatant was collected and stored at-20°C until further analysis.

### Cytokine analysis

Experimental method as outlined in Example 3.1 was modified in order to analyze GM-CSGF levels in human dermals fibroblasts stimulated with all-trans retinoic acid, retinol, arachidonic acid, β-aescin, and dodecahexaenoic acid (ethyl ester) at a range of concentrations. Experiments were performed in duplicates.

### Example 7.2 Results

Figure 8 shows inhibition of GM-CSF production in human dermal fibroblasts by gibberrellic acid, naringenin and N-acetyl aspartic acid (and demonstrating anti-pigmentation activity).

### Example 7:3 Methods

Full thickness skin from surgical waste material from breast reductions was collected. Subcutaneous fat was removed with a scalpel. 8 mm punch biopsies was taken from the skin and referred to as skin explants. Thereafter, the explants were put on

Millipore cell culture inserts (12mm in diameter). Inserts containing skin explants were put in 6-well plate (1 insert/well) and 1 mL of supplemented keratinocyte medium (Cascade Biologics) was added to each well to allow survival and nutrition of the explants. The skin explants were treated with 1 % w/w solutions of gibberellic acid, N-acetyl aspartic acid and naringenin in propyleneglycol for 24h, or with the benchmark control: tretinoin (Aberela® ex. Janssen) and the corresponding placebo. After pre-treatment the explants were UV-irradiated with 120mJ/cm² UVA and UVB and thereafter re-stimulated topically with gibberellic acid, naringenin and N-acetyl aspartic acid for 24 hours. Supernatants were thereafter collected and analyzed for GM-CSF production.

### Example 7:4 Results

Figure 9shows the effect on topical treatment of skin explants with 1 % w/w solution of gibberellic acid, naringenin and N-acetyl aspartic acid in propylene glycol on the suppression of UV-induced expression of GM-CSF.

Gibberellic acid, naringenin and N-acetylaspartic acid inhibit the paracrine production of the melanogenic factor GM-CSF stimulated by either PMA or UV in fibroblasts and skin explants. These results implicate the efficacy of the described ingredients in UV or inflammation-induced hyperpigmentation. Inhibition of GM-CSF production can reduce in reduction in skin pigmentation and UV-induced skin pigmentation, can reduce redness in the skin and provide a more even skin complexion.

**Example 8 VEGF-inhibition activity of naringenin, N-acetyl aspartic acid, gibberellic acid and β-aescin (COMBATTING REDNESS, BROKEN VEINS, PUFFY EYES)**

### Example 8.1 Method

Cell culture and cytokine analysis was carried out on human dermal fibroblasts as described in Example 3.1. The methodology was adapted in order analyze VEGF production.

### Example 8.2 Results

NA, GA and TripleA all reduce inflammatory induced VEGF synthesis in cultured dermal fibroblasts. In accordance with the known pro-inflammatory effect of RA an additional increase in VEGF could be detected in samples treated with RA together with the inflammatory inducer PMA. Treatment with retinol did not have that effect, is resulted a small but insignificant decrease in VEGF.Treatmentwith BE also resulted in a decreased VEGF expression, although insignificant.

Figure 10-shows the inhibition of inflammation induced VEGF by naringenin, N-acetyl aspartic acid, gibberellic acid and β-aescin.

Naringenin, N-acetyl aspartic acid, gibberellic acid and β-aescin inhibit VEGF activity and are therefore useful as angiogenesis inhibitors for prevention of associated unwanted features of skin aging such as anti-redness, broken veins and puffiness around the eyes.

**Example 9 Naringenin and docosahexaenoic acid (ethyl ester) stimulate keratin-6 production in normal human keratinocytes *[WOUND HEALING]***

### Example 9.1 Methods

Cells were cultured as described in Example 5.1. and the methodology described in Example 3.1 was adapted in order analyze keratin-6 production.

### Example 9.2 Results

Figure 11 shows stimulation of keratin 6 in human keratinocytes stimulated with compounds of the invention, retinoic acid (RA) and retinol (Rol) and the positive control calcium.

Compounds naringenin and docosahexaenoic acid (ethyl ester) stimulated keratin-6 production in normal human keratinocytes and can assist the process of wound healing and skin repair.

### Example 10 N-Acetylaspartic acid, docosahexaenoic acid (ethyl ester) and β-aescin.

### stimulate fibronectin production in normal human keratinocytes [WOUND HEALING]

### Example 10.1 Methods

Cells were cultured as described in Example 5.1. and the methodology described in Example 3.1 was adapted in order analyze fibronectin production.

### Example 10.2 Results

Figure 12 shows the stimulation of fibronectin in human keratinocytes stimulated with all-trans retinoic acid, retinol, compounds of the invention and the positive control calcium.

Compounds of the invention had superior effect on fibronectin release in human keratinocytes after 6 days stimulation compared to all-trans retinoic acid and retinol. Retinoic acid is a known activator of fibronectin in skin (Schwartz and Kligman 1995b)

### Example 11 Compounds gibberellic acid, naringenin and N-acetyl aspartic acid stimulate fibroblast growth factor (FGF) production in human skin explants [WOUND HEALING]

### Example 11.1 Methods

*Human skin explants were obtained and treated as described in Example 7.3.*

The methodology described in Example 3.1 was adapted in order analyze FGF production in skin explants.

### Example 11.2 Results

Figure 13 shows the stimulation of FGF production in human skin explants by compounds of the invention Stimulation of FGF production by naringenin, gibberellic acid and N-acetylaspartic acid in human skin explants suggests wound healing benefits from topical application of these compounds to the skin.

### Example 12 Stimulation of growth factors CTGF and HBEGF on the gene level in Affymetrix gene array by compounds of the invention

### Example 12.1 Methods

Skin explants were obtained as detailed in Example 3.1. Compounds naringenin and N-acetyl aspartic acid were topically applied on skin explants for 24 hours and investigated with Affymetrix gene array.

### Example 12.2 Results

| **ID** | **Gene Name** | **Gene Symbol** | **NG** | **Triple A** | **All-trans retinoic acid** |
|---|---|---|---|---|---|
| 209101_at | connective tissue growth factor | CTGF | 1,05 | 1,03 | No effect |
| 38037_at | heparin-binding EGF-like growth factor | HBEGF | 1,19 | No effect | 1,21 |

Table 3 shows the stimulatory action of naringenin and N-acetyl aspartic acid on CTGF expression and also naringenin's stimulatory effect on HBEGF (log2-ratio|>1).

Using a cut-off fold change |log2-ratio|>1 the analysis which was suggested by (Quackenbush J et al 2002) the Affymetrix gene array on skin explants showed that naringenin and N-acetyl aspartic acid induced CTGF. NG also stimulated the expression of HBEGF. It is noteworthy that all-trans retinoic acid did not have any effect on CTGF.

### Example 13 Gibberellic acid, naringenin, N-acetyl aspartic acid, arachidonic acid and docosahexaenoic acid (ethyl ester) stimulate Kallikrein 8 production in human normal keratinocytes (DESQUAMATION/COMPLEXION BRIGHTENING/ DEPIGMENTATION)

Kallikrein 8 is involved in the late differentiation of keratinocytes: the desquamation process. Kallikrein 8 is a biomarker for epidermal turnover and exfoliation.

### Example 13.1 Method

Keratinocytes used for Kallikrein 8 experiments were cultured as described in example 2.1. Experiments were performed in monolayers with 80% confluence. The cells were stimulated for 4 days prior to kallikrein 8 analysis with the different actives and then culture supernatant were collected and stored in -20°C until further analysis. Kallikrein 8 production was analysed in cell culture supernatants from stimulated keratinocytes using a ELISA assay specific for Kallikrein 8 (Uscn Life Science Inc, E90690Hu). Results were compared with cell culture supernatants from DMSO vehicle treated cells. The assays utilized a 96-well microplate format and were processed according to the manufacturer's protocol, including generation of a standard curve prepared in background assay diluents. Absorbance of the assay dye was read at a Synergy HT plate reader. Values that fell within the linear range of the calibration assay were accepted. Quantities were determined by comparison to standard curves obtained for KLK8.

### Example 13.2 Results

Figure 14 shows significant stimulation of kallikrein 8 by gibberellic acid, naringenin, N-acetyl aspartic acid, arachidonic acid and docosahexaenoic acid (ethyl ester), as well as positive controls retinol and retinoic acid.

Gibberellic acid, naringenin, N-acetyl aspartic acid, arachidonic acid and docosahexaenoic acid (ethyl ester) induced a significant increase in the synthesis of kallikrein 8 in human normal keratinocytes. The process of desquamation is essential in maintaining an intact skin barrier and for healthy looking skin.

Enhanced epidermal turnover and desquamation can result in exfoliation of excessive pigmentation and therefore contribute to combating skin darkening due to the presence of melanin in the skin and also result in improving skin lustre and/or brightness of the complexion.

### Example 14 Compounds of the invention stimulate Keratin 1/10 production in human normal keratinocytes

Cells were cultured as described in Example 5.1. and the methodology described in Example 3.1 was adapted in order analyze keratin 1/10 production.

### Example 14.2 Results

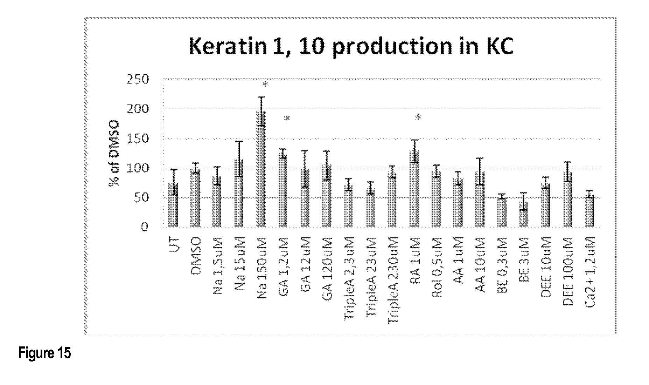

Figure 15 shows synthesis of keratin 1/10 expressed at % of vehicle control synthesis.

Naringenin (150 microM) and gibberellic acid (1.2 microM) induced a significant increase in the synthesis of Keratin1/10 in human normal keratinocytes. The process of keratinocyte differentiation is essential in maintaining an intact skin barrier and for healthy looking skin.

**Example 15 Compounds gibberellic acid, naringenin and N-acetyl aspartic acid inhibit genes involved in acne**

### exacerbation

Androgen stimulation is an important factor in adolescent and adult acne. Androgen causes an increased production of sebum (Thiboutot 2004). The Cmap ingredients showed anti-steroid 5-α-reductase expression and anti-hydroxysteroid (17-beta) dehydrogenase 2 (HSD17B2) activities in affymetrix gene arrays which implicates control of estrogen and androgen levels in the human skin. The therapeutic potential of this enzyme family in the treatment of Acne has extensively been described. (Poirier 2003)

### Example 15.1 Method

Skin explants were obtained as detailed in Example 3.1. Compounds gibberellic acid, naringenin and N-acetyl aspartic acid were topically applied on skin explants for 24 hours and investigated with Affymetrix gene array.

### Example 15.2 Results

**Table 1. Inhibition of genes involved in acne exacerbation**

| **ID** | **Gene Name** | **Gene Symbol** | **GA** | **NG** | **Triple A** | **Tretino in** |
|---|---|---|---|---|---|---|
| 238669_at | prostaglandin-endoperoxide synthase 1 (prostaglandin G/H synthase and cyclooxygenase) | PTGS1 | -1,11 | -3,51 | -0,20 | -1,03 |
| 204818_at | hydroxysteroid (17-beta) dehydrogenase 2 | HSD17B2 | -0,52 | -0,83 | -1,46 | -0,48 |
| 243444_at | steroid 5 alpha-reductase 3 | SRD5A3 | -0,75 | 1,25 | -1,54 | 0,29 |
| 206552_s_at | tachykinin, precursor 1 | TAC1 | -0,62 | -2,78 | -1,23 | -4,01 |
| 204446_s_at | arachidonate 5-lipoxygenase | ALOX5 | -1,01 | -0,69 | 0,15 | -0,69 |

Using a cut-off fold change |log2-ratio|>1 the analysis which was suggested by Quackenbush et al (Quackenbush 2002), the Affymetrix gene array on skin explants showed that several genes involved inflammation, sebum, androgen and neuropeptine stimulation were downregulated by the compounds of the invention. N-acetyl aspartic acid shows anti-steroid 5-α-reductase expression in affymetrix gene arrays and anti-hydroxysteroid (17-beta) dehydrogenase 2 (HSD17B2) activity. Gibberellic acid, naringenin and tretinoin all show strong anti-cyclooxygenase I (COXI) effect. A strong downregulation in TAC1 which encodes neurokinin A (substance P) was seen by naringenin and N-acteyl aspartic acid as well as tretinoin. Gibberellic acid showed anti-arachidonate 5-lipoxygenase activity.

Androgen stimulation is an important factor in adolescent and adult acne. Androgen causes an increased production of sebum (Thiboutot 2004). The Cmap ingredients showed anti-steroid 5-α-reductase expression and anti-hydroxysteroid (17-beta) dehydrogenase 2 (HSD17B2) activities in Affymetrix gene arrays which implicates control of estrogen and androgen levels in the human skin. The therapeutic potential of this enzyme family in the treatment of acne has extensively been described (Poirier 2003).

Regulation of neuropeptides plays an important role in the exacerbation of acne. Specifically, substance P play a role in the exacerbation of acne from a neurological point of view, where *in vitro* studies revealed that Substance P promotes both the proliferation and the differentiation of sebaceous glands (Toyoda et al. 2002). A strong downregulation in TAC1 which encodes neurokinin A (substance P) was seen by NG/ N-Triple A acid as well as tretinoin.

The Cmap ingredients showed gene regulation behavior linked to reduction of expression of enzymes involved in leukotriene and prostaglandin signalling pathways, i.e. arachidonate 5-lipoxygenase, cyclooxygenase which both have been shown to be evaluated in acne-involved facial skin (Alestas et al. 2006). It is noteworthy that cox inhibitors have been shown to be effective in the management of premenstrual acne.

We were also able to show that the Cmap ingredients reduce IL-6 production in dermal fibroblasts which indicates dermal anti-inflammatory action which is an important target to combat acne. For example, long-term treatment with Zileuton which directly targets reduced the content of neutral lipids and interleukin-6 release (Zouboulis et al. 2009).

### Example 16 Gibberellic acid, naringenin and N-acetyl aspartic acid are non-irritating to the skin

*In vitro* skin irritation studies carried out on pure gibberellic acid, naringenin and N-acetyl aspartic acid on human reconstructed epidermis (SkinEthic model) in accordance with the OECD guideline 439, found all compounds to be non-irritating to skin.

The studies, aimed at evaluating the capability of the test compound to induce skin irritation effects by a cytotoxicity assay on *in vitro* human reconstructed epidermis (SkinEthic), were carried out according to the ECVAM protocol of April 27^{th}, 2007, the B46 method of the official journal of the European union, dated August 14^{th} 2009 and the OECD guideline 439.

### Example 17 Oil-in-water cream, typical face cream product

| | % w/w |
|---|---|
| HYDROGENATED POLYISOBUTENE | 4.5 |
| CYCLOPENTASILOXANE | 4.5 |
| DIMETHICONE | 2.5 |
| GLYCERYLSTEARATE | 2 |
| CETYL ALCOHOL | 2 |
| PEG-40 STEARATE | 1.25 |
| SODIUM ACRYLATE/ACRYLOYLDIMETHYL TAURATE COPOLYMER | 1 |
| TOCOPHERYL ACETATE | 0.5 |
| PRESERVATIVES | QS |
| PERFUME | QS |
| *Compound of invention* | *1* |
| WATER | to 100 |

**Example 18 W/Si emulsion, a typical liquid foundation product**

| | %w/w |
|---|---|
| CYCLOPENTASILOXANE | 14 |
| PIGMENTS | 9 |
| POLYGLYCERYL-4ISOSTEARATE | 5 |
| ISODODECANE | 4 |
| BUTYLENE GLYCOL | 2 |
| BIS-PEG/PPG-14/14 DIMETHICONE | 2 |
| HYDROGENATED POLYISOBUTENE | 1.5 |
| BEESWAX | 1.2 |
| MAGNESIUM SULPHATE | 1 |
| PRESERVATIVES | QS |
| PERFUME | QS |
| *Compound of invention* | *0.5* |
| WATER | to 100 |

### Example 19 Hydroalcoholic gel, a typical serum-type face product

| Raw materials | % w/w |
|---|---|
| SILICONES | 3.0 |
| GLYCERIN | 3.0 |
| BUTYLENE GLYCOL | 2.0 |
| *Compound of invention* | ***2***.***0*** |
| THICKENER | 1.0 |
| PEG-40 HYDROGENATED CASTOR OIL | 0.5 |
| POLYMER | 0.2 |
| SODIUM HYALURONATE | 0.03 |
| PARFUM | QSP |
| TETRAHYDROXYPROPYL ETHYLENEDAMINE | QSP |
| PRESERVATIVES | QSP |
| WATER | To 100 |

It should be readily apparent to one of ordinary skill in the art that the examples disclosed herein below represent generalised examples only, and that other arrangements and methods capable of reproducing the invention are possible and are embraced by the present invention.

The words "comprises/comprising" and the words "having/including" when used herein with reference to the present invention are used to specify the presence of stated features, integers, steps or components but do not preclude the presence or addition of one or more otherfeatures, integers, steps, components orgroups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### Reference List

Abe, K., Butcher, R. W., Nicholson, W. E., Baird, C. E., Liddle, R. A., and Liddle, G. W. (1969). "Adenosine 3',5'-monophosphate (cyclic AMP) as the mediator of the actions of melanocyte stimulating hormone (MSH) and norepinephrine on the frog skin." Endocrinology, 84(2), 362-368.
Alestas, T., Ganceviciene, R., Fimmel, S., Muller-Decker, K., and Zouboulis, C. C. (2006). "Enzymes involved in the biosynthesis of leukotriene B4 and prostaglandin E2 are active in sebaceous glands." J. Mol. Med (Berl), 84(1), 75-87.
Bennett, M. F., Robinson, M. K., Baron, E. D., and Cooper, K. D. (2008). "Skin immune systems and inflammation: protector of the skin or promoter of aging?" J. Investig. Dermatol. Symp. Proc.,13(1),15-19.
Borgono, C. A., Gavigan, J. A., Alves, J., Bowles, B., Harris, J. L., Sotiropoulou, G., and Diamandis, E. P. (2007). "Defining the extended substrate specificity of kallikrein 1-related peptidases." Biol. Chem., 388(11),1215-1225.
Bratton, D. L., Hamid, Q., Boguniewicz, M., Doherty, D. E., Kailey, J. M., and Leung, D. Y. (1995). "Granulocyte macrophage colony-stimulating factor contributes to enhanced monocyte survival in chronic atopic dermatitis." J. Clin. Invest, 95(1), 211-218. Brauchle, M., Funk, J. O., Kind, P., and Werner, S. (1996a). "Ultraviolet B and H2O2 are potent inducers of vascular endothelial growth factor expression in cultured keratinocytes." J. Biol. Chem., 271(36), 21793-21797.
Brauchle, M., Funk, J. O., Kind, P., and Wemer, S. (1996b). "Ultraviolet B and H2O2 are potent inducers of vascular endothelial growth factor expression in cultured keratinocytes." J. Biol. Chem., 271(36), 21793-21797.
Busca, R., and Ballotti, R. (2000). "Cyclic AMP a key messenger in the regulation of skin pigmentation." Pigment Cell Res., 13(2), 60-69.
Cohen, T., Nahari, D., Cerem, L. W., Neufeld, G., and Levi, B. Z. (1996). "Interleukin 6 induces the expression of vascular endothelial growth factor." J. Biol. Chem., 271(2), 736-741.
Conner, E.M., and Grisham, M. B. (1996). "Inflammation, free radicals, and antioxidants." Nutrition, 12(4), 274-277.
Dasu, M. R., Barrow, R. E., Spies, M., and Hemdon, D. N. (2003). "Matrix metalloproteinase expression in cytokine stimulated human dermal fibroblasts." Bums, 29(6), 527-531.
Farage, M. A., Miller, K. W., Elsner, P., and Maibach, H. I. (2008). "Intrinsic and extrinsic factors in skin ageing: a review." Int. J. Cosmet. Sci., 30(2), 87-95.
Fisher, G. J., Wang, Z. Q., Datta, S. C., Varani, J., Kang, S., and Voorhees, J. J. (1997). "Pathophysiology of premature skin aging induced by ultraviolet light." N. Engl. J. Med, 337(20),1419-1428.
Fuchs, E. (2007). "Scratching the surface of skin development." Nature, 445(7130), 834-842.
Gillbro, J. M., and Olsson, M. J. (2011). "The melanogenesis and mechanisms of skin-lightening agents-existing and new approaches." Int. J. Cosmet. Sci., 33(3), 210-221.
Gosain, A., and DiPietro, L. A. (2004). "Aging and wound healing." World J. Surg., 28(3), 321-326.
Griffiths, C. E., Kang, S., Ellis, C. N., Kim, K. J., Finkel, L. J., Ortiz-Ferrer, L. C., White, G. M., Hamilton, T. A., and Voorhees, J. J. (1995). "Two concentrations of topical tretinoin (retinoic acid) cause similar improvement of photoaging but different degrees of irritation. A double-blind, vehicle-controlled comparison of 0.1 % and 0.025% tretinoin creams." Arch. Dermatol., 131 (9), 1037-1044.
Grossman, R. M., Krueger, J., Yourish, D., Granelli-Pipemo, A., Murphy, D. P., May, L. T., Kupper, T. S., Sehgal, P. B., and Gottlieb, A. B. (1989). "Interleukin 6 is expressed in high levels in psoriatic skin and stimulates proliferation of cultured human keratinocytes." Proc. Natl. Acad. Sci. U. S. A, 86(16), 6367-6371.
Hamilton, J. A. (2008). "Colony-stimulating factors in inflammation and autoimmunity." Nat. Rev. Immunol., 8(7), 533-544.
Hara, T., Miyazaki, M., Hakuno, F., Takahashi, S., and Chida, K. (2011). "PKCeta promotes a proliferation to differentiation switch in keratinocytes via upregulation of p27Kip1 mRNA through suppression of JNK/c-Jun signaling under stress conditions." Cell Death. Dis., 2, e157.
Hearing, V. J., and Jimenez, M. (1987). "Mammalian tyrosinase-the critical regulatory control point in melanocyte pigmentation." Int. J. Biochem., 19(12), 1141-1147.
Hirakawa, S., Fujii, S., Kajiya, K., Yano, K., and Detmar, M. (2005). "Vascular endothelial growth factor promotes sensitivity to ultraviolet B-induced cutaneous photodamage." Blood, 105(6), 2392-2399.
Hirobe, T., Furuya, R., Ifuku, O., Osawa, M., and Nishikawa, S. (2004). "Granulocyte-macrophage colony-stimulating factor is a keratinocyte-derived factor involved in regulating the proliferation and differentiation of neonatal mouse epidermal melanocytes in culture." Exp. Cell Res., 297(2), 593-606.
Hunt, G., Todd, C., Cresswell, J. E., and Thody, A. J. (1994). "Alpha-melanocyte stimulating hormone and its analogue Nle4DPhe7 alpha-MSH affect morphology, tyrosinase activity and melanogenesis in cultured human melanocytes." J. Cell Sci., 107 (Pt 1), 205-211.
Igarashi, A., Okochi, H., Bradham, D. M., and Grotendorst, G. R. (1993). "Regulation of connective tissue growth factor gene expression in human skin fibroblasts and during wound repair." Mol. Biol. Cell, 4(6), 637-645.
Im, S., Moro, O., Peng, F., Medrano, E. E., Cornelius, J., Babcock, G., Nordlund, J. J., and Abdel-Malek, Z. A. (1998). "Activation of the cyclic AMP pathway by alpha-melanotropin mediates the response of human melanocytes to ultraviolet B radiation." Cancer Res., 58(1), 47-54.
Imokawa, G., Yada, Y., Kimura, M., and Morisaki, N. (1996). "Granulocyte/macrophage colony-stimulating factor is an intrinsic keratinocyte-derived growth factor for human melanocytes in UVA-induced melanosis." Biochem. J., 313 (Pt 2), 625-631.
Infanger, M., Schmidt, O., Kossmehl, P., Grad, S., Ertel, W., and Grimm, D. (2004). "Vascular endothelial growth factor serum level is strongly enhanced after bum injury and correlated with local and general tissue edema." Bums, 30(4), 305-311.
Jabbour, S. A. (2003). "Cutaneous manifestations of endocrine disorders: a guide for dermatologists." Am. J. Clin. Dermatol., 4(5), 315-331.
Jimbow, K., Alena, F., Dixon, W., and Hara, H. (1992). "Regulatory factors of pheo- and eumelanogenesis in melanogenic compartments." Pigment Cell Res., Suppl 2, 36-42.
Kahari, V. M., and Saarialho-Kere, U. (1997a). "Matrix metalloproteinases in skin." Exp. Dermatol., 6(5),199-213.
Kahari, V. M., and Saarialho-Kere, U. (1997b). "Matrix metalloproteinases in skin." Exp. Dermatol., 6(5),199-213.
Kerkvliet, E. H., Docherty, A. J., Beertsen, W., and Everts, V. (1999). "Collagen breakdown in soft connective tissue explants is associated with the level of active gelatinase A (MMP-2) but not with collagenase." Matrix Biol., 18(4), 373-380.
Kessler-Becker, D., Smola, S., Krieg, T., and Eckes, B. (2004). "High plasminogen activator inhibitor type 2 expression is a hallmark of scleroderma fibroblasts in vitro." Exp. Dermatol., 13(11), 708-714.
Kishibe, M., Bando, Y., Terayama, R., Namikawa, K., Takahashi, H., Hashimoto, Y., Ishida-Yamamoto, A., Jiang, Y. P., Mitrovic, B., Perez, D., lizuka, H., and Yoshida, S. (2007). "Kallikrein 8 is involved in skin desquamation in cooperation with other kallikreins." J. Biol. Chem., 282(8), 5834-5841.
Knott, A., Drenckhan, A., Reuschlein, K., Lucius, R., Doring, O., Bottger, M., Stab, F., Wenck, H., and Gallinat, S. (2010). "Decreased fibroblast contractile activity and reduced fibronectin expression are involved in skin photoaging." J. Dermatol. Sci., 58(1), 75-77.
Komatsu, N., Saijoh, K., Toyama, T., Ohka, R., Otsuki, N., Hussack, G., Takehara, K., and Diamandis, E. P. (2005). "Multiple tissue kallikrein mRNA and protein expression in normal skin and skin diseases." Br. J. Dermatol., 153(2), 274-281.
Kossakowska, A. E., Edwards, D. R., Prusinkiewicz, C., Zhang, M. C., Guo, D., Urbanski, S. J., Grogan, T., Marquez, L. A., and Janowska-Wieczorek, A. (1999). "Interleukin-6 regulation of matrix metalloproteinase (MMP-2 and MMP-9) and tissue inhibitor of metalloproteinase (TIMP-1) expression in malignant non-Hodgkin's lymphomas." Blood, 94(6), 2080-2089.
Lamb, J., Crawford, E. D., Peck, D., Modell, J. W., Blat, I. C., Wrobel, M. J., Lerner, J., Brunet, J. P., Subramanian, A., Ross, K. N., Reich, M., Hieronymus, H., Wei, G., Armstrong, S. A., Haggarty, S. J., Clemons, P. A., Wei, R., Carr, S. A., Lander, E. S., and Golub, T. R. (2006). "The Connectivity Map: using gene-expression signatures to connect small molecules, genes, and disease." Science, 313(5795), 1929-1935.
LERNER, A. B., and MCGUIRE, J. S. (1961). "Effect of alpha- and betamelanocyte stimulating hormones on the skin colour of man." Nature, 189,176-179.
LERNER, A. B., and MCGUIRE, J. S. (1964). "MELANOCYTE-STIMULATING HORMONE AND ADRENOCORTICOTROPHIC HORMONE. THEIR RELATION TO PIGMENTATION." N. Engl. J. Med, 270,539-546. LERNER, A. B., SHIZUME, K., FITZPATRICK, T. B., and Mason, H. S. (1954). "MSH: the melanocyte-stimulating hormone." AMA. Arch. Derm. Syphilol., 70(5), 669-674.
Levine, N., Sheftel, S. N., Eytan, T., Dorr, R. T., Hadley, M. E., Weinrach, J. C., Ertl, G. A., Toth, K., McGee, D. L., and Hruby, V. J. (1991). "Induction of skin tanning by subcutaneous administration of a potent synthetic melanotropin." JAMA, 266(19), 2730-2736.
Lu, B., Rothnagel, J. A., Longley, M. A., Tsai, S. Y., and Roop, D. R. (1994). "Differentiation-specific expression of human keratin 1 is mediated by a composite AP-1/steroid hormone element." J. Biol. Chem., 269(10), 7443-7449.
Meier, B., Radeke, H. H., Selle, S., Younes, M., Sies, H., Resch, K., and Habermehl, G. G. (1989). "Human fibroblasts release reactive oxygen species in response to interleukin-1 or tumour necrosis factor-alpha." Biochem. J., 263(2), 539-545.
Parks, W. C., Wilson, C. L., and Lopez-Boado, Y. S. (2004). "Matrix metalloproteinases as modulators of inflammation and innate immunity." Nat. Rev. Immunol., 4(8), 617-629.
Phelps, K. R. (1990). "Edema.".
Poirier, D. (2003). "Inhibitors of 17 beta-hydroxysteroid dehydrogenases." Curr. Med. Chem., 10(6), 453-477.
Proksch, E., Brandner, J. M., and Jensen, J. M. (2008). "The skin: an indispensable barrier." Exp. Dermatol., 17(12), 1063-1072. Quackenbush, J. (2002). "Microarray data normalization and transformation." Nat. Genet., 32 Suppl, 496-501.
Rijken, F., and Bruijnzeel, P. L. (2009). "The pathogenesis of photoaging: the role of neutrophils and neutrophil-derived enzymes." J. Investig. Dermatol. Symp. Proc., 14(1), 67-72.
Salven, P., Anttonen, K., Repo, H., Joensuu, H., and Orpana, A. (2001). "Endotoxins induce and interferon alpha suppresses vascular endothelial growth factor (VEGF) production in human peripheral blood mononuclear cells." FASEB J., 15(7), 1318-1320.
Sandjeu, Y., and Haftek, M. (2009). "Desmosealin and other components of the epidermal extracellular matrix." J. Physiol Pharmacol., 60 Suppl 4, 23-30.
Schauer, E., Trautinger, F., Kock, A., Schwarz, A., Bhardwaj, R., Simon, M., Ansel, J. C., Schwarz, T., and Luger, T. A. (1994). "Proopiomelanocortin-derived peptides are synthesized and released by human keratinocytes." J. Clin. Invest, 93(5), 2258-2262. Schonthaler, H. B., Huggenberger, R., Wculek, S. K., Detmar, M., and Wagner, E. F. (2009). "Systemic anti-VEGF treatment strongly reduces skin inflammation in a mouse model of psoriasis." Proc. Natl. Acad. Sci. U. S. A, 106(50), 21264-21269.
Schwartz, E., and Kligman, L. H. (1995a). "Topical tretinoin increases the tropoelastin and fibronectin content of photoaged hairless mouse skin." J. Invest Dermatol., 104(4), 518-522.
Schwartz, E., and Kligman, L. H. (1995b). "Topical tretinoin increases the tropoelastin and fibronectin content of photoaged hairless mouse skin." J. Invest Dermatol., 104(4), 518-522.
Secker, G. A., Shortt, A. J., Sampson, E., Schwarz, Q. P., Schultz, G. S., and Daniels, J. T. (2008). "TGFbeta stimulated re-epithelialisation is regulated by CTGF and Ras/MEK/ERK signaling." Exp. Cell Res., 314(1), 131-142.
Shirakata, Y., Kimura, R., Nanba, D., Iwamoto, R., Tokumaru, S., Morimoto, C., Yokota, K., Nakamura, M., Sayama, K., Mekada, E., Higashiyama, S., and Hashimoto, K. (2005). "Heparin-binding EGF-like growth factor accelerates keratinocyte migration and skin wound healing." J. Cell Sci., 118(Pt 11), 2363-2370.
Smith, J. R., Lanier, V. B., Braziel, R. M., Falkenhagen, K. M., White, C., and Rosenbaum, J. T. (2007). "Expression of vascular endothelial growth factor and its receptors in rosacea." Br. J. Ophthalmol., 91(2), 226-229.
Thiboutot, D. (2004). "Regulation of human sebaceous glands." J. Invest Dermatol., 123(1),1-12.
Thody, A. J., and Graham, A. (1998). "Does alpha-MSH have a role in regulating skin pigmentation in humans?" Pigment Cell Res., 11(5), 265-274.
Tiano, H. F., Loftin, C. D., Akunda, J., Lee, C. A., Spalding, J., Sessoms, A., Dunson, D. B., Rogan, E. G., Morham, S. G., Smart, R. C., and Langenbach, R. (2002). "Deficiency of either cyclooxygenase (COX)-1 or COX-2 alters epidermal differentiation and reduces mouse skin tumorigenesis." Cancer Res., 62(12), 3395-3401.
Toulza, E., Mattiuzzo, N. R., Galliano, M. F., Jonca, N., Dossat, C., Jacob, D., de, D. A., Wincker, P., Serre, G., and Guerrin, M. (2007). "Large-scale identification of human genes implicated in epidermal barrier function." Genome Biol., 8(6), R107.
Toyoda, M., Nakamura, M., Makino, T., Kagoura, M., and Morohashi, M. (2002). "Sebaceous glands in acne patients express high levels of neutral endopeptidase." Exp. Dermatol., 11(3), 241-247.
Trompezinski, S., Pernet, I., Schmitt, D., and Viac, J. (2001). "UV radiation and prostaglandin E2 up-regulate vascular endothelial growth factor (VEGF) in cultured human fibroblasts." Inflamm. Res., 50(8), 422-427.
Winrow, V. R., Winyard, P. G., Morris, C. J., and Blake, D. R. (1993). "Free radicals in inflammation: second messengers and mediators of tissue destruction." Br. Med Bull., 49(3), 506-522.
Wlaschek, M., Bolsen, K., Herrmann, G., Schwarz, A., Wilmroth, F., Heinrich, P. C., Goerz, G., and Scharffetter-Kochanek, K. (1993). "UVA-induced autocrine stimulation of fibroblast-derived-collagenase by IL-6: a possible mechanism in dermal photodamage?" J. Invest Dermatol., 101(2), 164-168.
Zouboulis, C. C., Fischer, T. C., Wohlrab, J., Barnard, J., and Alio, A. B. (2009). "Study of the efficacy, tolerability, and safety of 2 fixed-dose combination gels in the management of acne vulgaris." Cutis, 84(4), 223-229.
Alternative expressions of the inventive concept are set out in the following clauses:

### Clauses

1. Non-therapeutic cosmetic or dermatological use of at least one of a compound selected from the group consisting of:
   gibberellic acids, naringenins, N-acetylaspartic acids, β-aescin, arachadonic acid, quercetin, vitexin and docosahexaenoic acid (ethyl ester).
2. Use according to clause 1 wherein the use is in the treatment of, and/or prevention of, at least one sign of skin ageing or at least one sign of a skin damage condition associated with ageing, wherein the sign of skin ageing or skin damage is present on skin of the face, body or the scalp of a subject.
3. Use according to clause 2 wherein the at least one sign of skin ageing or the skin damage condition is produced by intrinsic biological aging (natural ageing) or photo-induced ageing (actinic ageing) caused by, for example, exposure to UV light, pollution or stress.
4. Use according to clause 3 wherein the at least one sign of skin ageing is chosen from wrinkles, fine lines, wizened skin, skin laxity associated with collagen loss or destruction, loss or reduction in skin integrity, lack of skin elasticity, lack of skin tone, thinned skin, sagging skin, skin suffering from degradation of collagen fibres, flaccid skin, and skin suffering from internal degradation.
5. Use according to clause 3 wherein the skin damage condition involves at least one of: inflammation, broken veins, redness, blotchiness, puffy eyes or dark circles, or skin pigmentation disorders.
6. Use according to clause 1 for skin lightening, for improving wound healing, for promoting evenness of skin tone by reducing skin redness or inflammation or UV induced skin redness, blotchiness or inflammation, for lightening (whitening) the colour of the skin or scalp, for promoting skin regeneration to produce more homogenous, firmer, more toned and more elastic skin, for promoting cell longevity, for promoting skin brightness, for promoting skin texture and tone uniformity and/or for reducing the appearance of skin pigmentation and/or skin darkening, or for the treatment or prevention of ulcerated areas or areas of cutaneous stress or damage brought about by exposure to UV or exposure to an irritant product, for enhancing skin desquamation, improving skin hydration, improving skin lustre and brightness or treatment or reduction of acne or other skin blemishes.
7. Use according to any preceding clause wherein at least one of the compounds is formulated into a cosmetically acceptable composition for topical application to the skin, body and/or scalp.
8. Use according to any one of clauses 1 to 7 wherein the compound is naringenin.
9. Use according to any one of clauses 1 to 7 wherein the compound is gibberellic acid.
10. Use according to any one of clauses 1 to 7 wherein the compound is N-acetylaspartic acid.
11. Cosmetic or dermatological composition comprising, in a physiologically acceptable medium, at least one of, or a combination of, compound(s) selected from the group consisting of: gibberellic acids, naringenins, N-acetylaspartic acids, β-aescin, arachadonic acid, quercetin, vitexin and docosahexaenoic acid (ethyl ester).
12. Composition according to clause 11 further comprising excipients suitable for topical application to the skin, body and/or scalp.
13. Composition according to clause 11 or 12 further comprising at least one of a sunscreen or UV filter(s), depigmenting agents for lightening the skin tone of a subject, moisturizing agents or further cosmetic agents or anti-aging components.
14. Composition of clause 13 wherein the further cosmetically active agent is a retinoid and/or derivative thereof, depigmenting agent, organic photoprotective agent and/or at least one inorganic photoprotective agent, peeling agents, antioxidants, moisturizing agents, etc. and mixtures thereof.
15. Composition according to clause 14 wherein the retinoid is selected from retinol, retinal, retinoic acid, an ester of retinol and an acid, C2-C20, such as propionate, acetate, linoleate, or retinol palmitate, and wherein the compound is not naringenin or quercetin.
16. Composition according to Clause 14 wherein the sunscreen or UV filters are present in an amount of from about 0.1% w/w to about 30% w/w, based on the total weight of the topical cosmetic composition.
17. Composition of clause 11 to 16 wherein the composition is provided in the form of:
   (i) a care, treatment, cleansing or protective product for skin, including the face and/or scalp;
   (ii) (ii) an anti-wrinkle or anti-ageing composition for the face;
   (iii) a composition for irritated skin;
   (iv) (iv) an anti-sun damage composition;
   (v) An anti-acne composition
   (v) an artificial tanning composition for the face and/or body; or
   (vi) an after-sun care composition.
19. Composition of any one of Clause 11 to 16 in the use of any one of Clauses 1 to 10.
20. Non-therapeutic cosmetic or dermatological procedure to prevent and/or delay and/or limit and/or treat at least one sign of skin ageing or a skin damage condition resulting from intrinsic or photo-induced ageing, comprising the step of applying to the skin, body and/or scalp of a subject an effective amount of the at least one compound of or composition of any one of clauses 1 to 19.
21. The procedure of clause 20, wherein application to the skin, body and/or scalp
   (i) lightens the colour of the skin or scalp of a subject, by preventing the formation and/or removing brown pigment blemishes and/or age blemishes and/or freckles;
   (ii) reduces at least one of skin redness, blotchiness or inflammation;
   (iii) reduces and/or smoothes skin imperfections such as wrinkles and/or fine lines;
   (iv) produces more homogenous, firmer, more toned and more elastic skin;
   (v) promotes wound healing;
   (vi) reduces puffy eyes and/or dark circles;
   (vii) treats acne, blemishes; or
   (viii) protects against UV-photo induced damage or degradation of collagen.
22. A composition or use substantially as described herein with reference to the accompanying examples and figures.

## Claims

1. An N-acetylaspartic acid having the general structure: 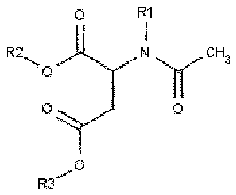 wherein R1 is hydrogen, or an unsubstituted, branched or unbranched, unsaturated or saturated C1-C6 alkyl, allyl, or aryl group; R2 is hydrogen, or a substituted or unsubstituted, branched or unbranched, unsaturated or saturated C1-C6 alkyl, allyl or aryl group; R3 is hydrogen, or a substituted or unsubstituted, branched or unbranched, unsaturated or saturated C1-C6 alkyl, allyl or aryl group, for use in the treatment of, and/or prevention of, at least one sign of skin ageing or at least one sign of a skin damage condition produced by photo-induced ageing (actinic ageing), for improving wound healing, or for treating psoriasis.

2. An N-acetyl aspartic acid for use according to claim 1, wherein the photo-induced aging is caused by exposure to UV light, pollution or stress.

3. An N-acetyl aspartic acid for use according to claim 1 or claim 2, wherein the at least one sign of skin ageing is chosen from wrinkles, fine lines, wizened skin, skin laxity associated with collagen loss or destruction, loss or reduction in skin integrity, lack of skin elasticity, lack of skin tone, thinned skin, sagging skin, skin suffering from degradation of collagen fibres, flaccid skin, and skin suffering from internal degradation.

4. An N-acetyl aspartic acid for use according to claim 1 or claim 2, wherein the skin damage condition involves at least one of: inflammation, broken veins, redness, blotchiness, puffy eyes or dark circles, or skin pigmentation disorders.

5. An N-acetyl aspartic acid for use according to any preceding claim, wherein the N-acetyl aspartic acid is N-acetyl-L-aspartic acid.

6. A composition comprising an N-acetyl aspartic acid having the general structure: 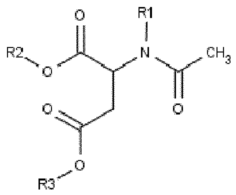 wherein R1 is hydrogen, or an unsubstituted, branched or unbranched, unsaturated or saturated C1-C6 alkyl, allyl, or aryl group; R2 is hydrogen, or a substituted or unsubstituted, branched or unbranched, unsaturated or saturated C1-C6 alkyl, allyl or aryl group; R3 is hydrogen, or a substituted or unsubstituted, branched or unbranched, unsaturated or saturated C1-C6 alkyl, allyl or aryl group for use in the treatment of, and/or prevention of, at least one sign of skin ageing or at least one sign of a skin damage condition produced by photo-induced ageing (actinic ageing), for improving wound healing, or for treating psoriasis.

7. The composition according to claim 6, further comprising excipients suitable for topical application to the skin, body and/or scalp.

8. The composition according to claim 6 or claim 7, further comprising at least one of a sunscreen or UV filter(s), depigmenting agents for lightening the skin tone of a subject, moisturizing agents or further cosmetic agents or anti-aging components.

9. The composition of claim 8, wherein the further cosmetically active agent is a retinoid and/or derivative thereof, depigmenting agent, organic photoprotective agent and/or at least one inorganic photoprotective agent, peeling agents, antioxidants, moisturizing agents, etc. and mixtures thereof.

10. The composition according to claim 9, wherein the retinoid is selected from retinol, retinal, retinoic acid, an ester of retinol and an acid, C2-C20, such as propionate, acetate, linoleate, or retinol palmitate.

11. The composition according to claim 9, wherein the sunscreen or UV filters are present in an amount of from about 0.1 % w/w to about 30% w/w, based on the total weight of the topical cosmetic composition.

12. The composition of any of claims 6 to 11, wherein the composition is provided in the form of:
(i) a care, treatment, cleansing or protective product for skin, including the face and/or scalp;
(ii) an anti-wrinkle or anti-ageing composition for the face;
(iii) a composition for irritated skin;
(iv) an anti-sun damage composition;
(v) an anti-acne composition
(v) an artificial tanning composition for the face and/or body; or
(vi) an after-sun care composition.

13. The composition according to any of claims 6 to 13, wherein the composition comprises 1% w/w N-acetylaspartic acid.

14. The composition according to any of claims 6 to 12, wherein the N-acetyl aspartic acid is N-acetyl-L-aspartic acid.

15. An N-acetyl aspartic acid for use according to any of claims 1 to 5 or the composition for use according to any of claims 6 to 14, wherein the use is in combination with at least one compound selected from the group consisting of: gibberellic acids, naringenins, β-aescin, arachadonic acid, quercetin, vitexin and docosahexaenoic acid (ethyl ester).
